# EUROPEAN PATENT SPECIFICATION

(11) **EP 3 191 598 B1**
(45) Date of publication and mention of the grant of the patent: **19.06.2019**
(21) Application number: 15760467.9
(22) Date of filing: 10.09.2015
(51) Int. Cl.: C12P 19/18, C13K 1/02, C12P 19/02, C07H 3/04, C12P 19/12, C13K 11/00, C12N 9/10

(54) **PROCESS FOR THE ENZYMATIC PREPARATION OF A PRODUCT GLUCOSIDE AND OF A CO-PRODUCT FROM AN EDUCT GLUCOSIDE**
VERFAHREN ZUR ENZYMATISCHEN HERSTELLUNG EINES PRODUKT-GLUCOSIDS UND EINES CO-PRODUKTS AUS EINEM EDUKT-GLUCOSID
PROCÉDÉ DE FABRICATION ENZYMATIQUE D'UN GLUCOSIDE DE PRODUIT ET D'UN PRODUIT COUPLÉ À PARTIR D'UN GLUCOSIDE D'ÉDUIT

(30) Priority: 10.09.2014 EP 14184302; 10.09.2014 EP 14184301; 29.10.2014 EP 14190891
(43) Date of publication of application: 19.07.2017
(73) Proprietor: Pfeifer & Langen GmbH & Co. KG, 50858 Köln (DE)
(72) Inventor: KOCH, Timo Johannes, 50189 Elsdorf (DE); HÄSSLER, Thomas, 50968 Köln (DE); KIPPING, Florian, 41542 Dormagen (DE)
(74) Representative: Kutzenberger Wolff & Partner
(86) International application number: PCT/EP2015/070724
(87) International publication number: WO 2016/038142

(56) References cited:
- EP-A1- 0 423 768
- WO-A1-2012/109274
- WO-A1-2014/060452
- US-A1- 2007 092 949
- SUZUKI M ET AL: "Synthesis of cellobiose from starch by the successive actions of two phosphorylases", NEW BIOTECHNOLOGY, ELSEVIER BV, NL, vol. 26, no. 3-4, 31 October 2009 (2009-10-31), pages 137-142, XP026741804, ISSN: 1871-6784, DOI: 10.1016/J.NBT.2009.07.004 [retrieved on 2009-07-22] cited in the application
- DESMET TOM ET AL: "Enzymatic glycosylation of small molecules: challenging substrates require tailored catalysts.", CHEMISTRY (WEINHEIM AN DER BERGSTRASSE, GERMANY) 27 AUG 2012, vol. 18, no. 35, 27 August 2012 (2012-08-27), pages 10786-10801, XP002734031, ISSN: 1521-3765
- MICHIYO YANASE ET AL: "[alpha]-Glucan phosphorylase and its use in carbohydrate engineering", JOURNAL OF THE SCIENCE OF FOOD AND AGRICULTURE, vol. 86, no. 11, 1 January 2006 (2006-01-01), pages 1631-1635, XP055103494, ISSN: 0022-5142, DOI: 10.1002/jsfa.2513
- CHRISTIANE LULEY-GOEDL ET AL: "Carbohydrate synthesis by disaccharide phosphorylases: Reactions, catalytic mechanisms and application in the glycosciences", BIOTECHNOLOGY JOURNAL, vol. 5, no. 12, 10 December 2010 (2010-12-10), pages 1324-1338, XP55232282, DE ISSN: 1860-6768, DOI: 10.1002/biot.201000217
- BERND NIDETZKY ET AL: "Cellobiose phosphorylase from Cellulomonas uda: gene cloning and expression in Escherichia coli, and application of the recombinant enzyme in a 'glycosynthase-type' reaction", JOURNAL OF MOLECULAR CATALYSIS B: ENZYMATIC, vol. 29, no. 1-6, 1 June 2004 (2004-06-01) , pages 241-248, XP055171347, ISSN: 1381-1177, DOI: 10.1016/j.molcatb.2003.11.014
- MANU R M DE GROEVE ET AL: "Engineering of cellobiose phosphorylase for glycoside synthesis", JOURNAL OF BIOTECHNOLOGY, ELSEVIER SCIENCE PUBLISHERS, AMSTERDAM, NL, vol. 156, no. 4, 11 July 2011 (2011-07-11) , pages 253-260, XP028119258, ISSN: 0168-1656, DOI: 10.1016/J.JBIOTEC.2011.07.006 [retrieved on 2011-07-20]
- SEIBEL J ET AL: "GLYCOSYLATION WITH ACTIVATED SUGARS USING GLYCOSYLTRANSFERASES AND TRANSGLYCOSIDASES", BIOCATALYSIS AND BIOTRANSFORMATION, HARWOOD ACADEMIC PUBL., BASEL, CH, vol. 24, no. 5, 1 January 2006 (2006-01-01), pages 311-342, XP008073465, ISSN: 1024-2422, DOI: 10.1080/10242420600986811

## Description

The invention relates to a process for the preparation of a product glucoside, i.e. an oligosaccharide, namely cellobiose, and of a co-product, i.e. a monosaccharide, namely fructose, from an educt glucoside, i.e. from an oligosaccharide, namely from sucrose, with enzymatic catalysis. The educt glucoside is thereby first cleaved enzymatically to glucose 1-phosphate and the co-product, and the glucose 1-phosphate is subsequently reacted to give the product glucoside. A reactant for the glucose 1-phosphate is added, i.e. a further saccharide, namely glucose. The co-product formed in the cleavage of the educt glucoside, and the product glucoside formed in the reaction of the glucose 1-phosphate are preferably each isolated, or alternatively, the co-product is further converted to a converted co-product which is then isolated.

Carbohydrates can exhibit diverse advantageous properties *inter alia* in the field of human and animal nutrition. The disaccharide cellobiose in particular is also distinguished by a potentially prebiotic action and a combination, rare for disaccharides, of low solubility, sweetness and hygroscopicity. This opens up diverse possible applications as a functional ingredient in foodstuffs and feeds. Other glycosides, such as, for example, glycosyl glycerol, are suitable for use in cosmetics. Starting from disaccharides that occur ubiquitously in nature, such as sucrose, lactose, mannose and maltose, other disaccharides can be prepared enzymatically. An important intermediate thereby is glucose 1-phosphate, which is obtainable from the glucose-containing disaccharides by means of a suitable phosphorylase with addition of a phosphate source. The glucose 1-phosphate can selectively be reacted with a suitable reactant, with catalysis by means of a further phosphorylase, to give further products.

Cellulose as a starting material for the chemical preparation of cellobiose is known from the prior art (A.N. Pereira et al., Preparation of cellodextrins in: Methods Enzymol., (1988), 160; US 2009/0281305). Cellobiose can be obtained enzymatically by hydrolysis of cellulose with cellulases, wherein glucose is formed by secondary activities and the degree of degradation is not complete (US 4,908,311, WO 2012/001102). A disadvantage of these processes is the incomplete degree of degradation of the cellulose, so that the product contains proportions of oligomers or longer-chained residues of cellulose. In the enzymatic process in particular, it is a disadvantage that most of the cellulases used exhibit beta-glycosidase activities and thus cleave a portion of the product to glucose. Complete conversions cannot be achieved in that manner.

US 4,908,311 discloses the working up of a product composition which comprises cellobiose obtained from cellulose by degradation, and glucose. The working up is carried out by means of immobilized yeast, whereby the glycose is to be metabolized completely by the yeast. However, the use of yeasts and the metabolization of a monosaccharide for working up a product introduce undefined impurities into the product as a result of the yeast. In addition, a valuable carbohydrate is lost through metabolization of the glucose, and the yield falls considerably.

Starch is also known from the prior art as a starting material for the preparation of cellobiose (M. Suzuki et al., Synthesis of cellobiose from starch by the successive actions of two phosphorylases in: New Biotechnology, (2009), 26, 3/4). In a two-stage process, following degradation of the starch, phosphate is separated from an intermediate product mixture, which comprises glucose 1-phosphate, by means of electrodialysis. During the second reaction stage, phosphate is separated off by precipitation as Mg(NH₄)PO₄. However, the use of starch as a starting material leads to secondary products in the form of non-degraded starch residues. The glucan phosphorylases used are able to degrade the starch chains to only a certain residual chain length even after debranching, for which reason this type of secondary products is intrinsic. Owing to the low solubility of starches in water, it is generally possible to work only to concentrations of 1% substrate, which leads to poor space-time yields of a production process and to the necessity to remove large amounts of water after synthesis of the glucose 1-phosphate (intermediate product).

JP-A 2009/060922 discloses the synthesis of glucose 1-phosphate from sucrose with a sucrose phosphorylase from *Leuconostoc mesenteroides.* The synthesis is carried out in a whole-cell fermentation with addition of sucrose and phosphoric acid, or phosphate salts. The concentration of the substrates is from 0.6 to 1.2 M. In a whole-cell fermentation, in addition to the actual product, numerous secondary products are also formed, which increase the outlay in terms of product isolation significantly. Furthermore, the process is complex to carry out because the growth of the microorganisms must first be stimulated until a specific biomass concentration is reached before the conditions for optimum product production can be established. Because of processes that consume glucose 1-phosphate in cells, yields in whole-cell transformations are lower than in pure enzyme processes.

EP-A 423 768 discloses a process for the preparation of cellobiose from sucrose using the three enzymes sucrose phosphorylase, glucose isomerase and cellobiose phosphorylase. As well as yielding glucose 1-phosphate, the treatment of sucrose with sucrose phosphorylase in the presence of phosphate also yields fructose, which is isomerized to glucose by means of glucose isomerase. Finally, the reaction of glucose 1-phosphate with the glucose obtained by isomerization, with catalysis by means of the cellobiose phosphorylase, yields cellobiose. The medium is buffered by imidazole. Starting material (sucrose) is employed at low concentrations of 100 mM and 200 mM only and product (cellobiose) is obtained at low concentrations of 147 mM only. After the reaction, the cellobiose phosphorylase is inactivated by heat, or the enzymes are separated from one another by immobilization in separate columns that are connected in series. The use of three enzymes is comparatively complex and results in losses in conversion. In addition, the isomerization of fructose to glucose is uneconomic. When three enzymes are used simultaneously in one system, it is not possible to take individual account of the optimum reaction conditions for each reaction stage. In the second reaction stage in particular, the increasing phosphate concentration interferes with the reaction. Therefore, in the third reaction stage too, the separation of cellobiose synthesis and phosphate removal is also disadvantageous in terms of the reaction equilibrium and the rate of reaction. The overall process is very slow and thus requires many hours in order to obtain satisfactory yields.

WO 2014/060452 relates to the identification, recombinant expression and characterization of a sucrose and sucrose-6'-phosphate phosphorylase of the thermophilic bacterium *Thermoanaerobacterium thermo-saccharolyticum.* This enzyme is said to be useful for the industrial production of D-fructose, D-fructose-6-phosphate, alpha-D-glucose-1-phosphate or alpha-D-glucosides. Reaction is performed in a two-phase system using water as first phase and ethyl acetate as second phase.

In the preparation of cellobiose in the presence of three enzymes, it has been described to crystallize out the cellobiose in order to increase the yield. This can take place either following the reaction, in which case the focus is on the recycling of the enzymes (H. Taniguchi et al., Enzymatic Synthesis of Cellobiose from Low Cost Carbohydrates; Biocatalysis and Biotechnology for functional foods and industrial products, 2007, AOCS Press, London), or at the same time as the reaction, as a result of which the position of the reaction equilibrium is especially to be shifted to a higher product yield (M. Kitaoka et al., Carbohydrate-Processing Phosphorolytic Enzymes in: Trends in Glycoscience and Glycotechnology, (2002), 14, 75). However, because cellobiose is a disaccharide which crystallizes particularly poorly, crystallization can only be achieved with reaction parameters that are disadvantageous for the actual reaction. For example, high supersaturations are also required in addition to low temperatures. The space-time yields are therefore very low.

S. Suzuki et al., New Biotechnology, Elsevier BV, NL, Vol. 26, No. 3-4, pages 137-142 relates to the synthesis of cellobiose from starch by the successive actions of two phosphorylases. The medium is buffered by 4-morpholinepropanesulfonic acid (MOPS) and contains magnesium acetate. High yields of cellobiose require long reaction times of 70 hours and more. The obtained cellobiose contains substantial amounts of phosphate.

The processes known from the prior art for the preparation of glycosides from other glycosides are not satisfactory in every respect and there is a need for improved processes. The improved processes are to be distinguished by a high space-time yield, efficient raw material utilization, an efficient use of enzymes, minimal consumption of raw materials and auxiliary substances, and high product purity. Secondary products, such as, for example, non-degraded polymer chains of the starting material (cellulose, starch, etc.), are to be suppressed or even avoided completely.

Accordingly, it is an object of the invention to provide a process for the preparation of glycosides from other glycosides which has advantages over the processes of the prior art.

The process according to the invention is to make optimum use, where possible, of the educts employed with regard to their intrinsic value, and is to achieve a high space-time yield. High long-term stability of the enzymes is thereby to be achieved. Where oligosaccharides comprising saccharide structural units of different intrinsic values are used as educts, where possible only the less intrinsically valuable saccharide structural units are to be used for the further synthesis and reacted further to give more intrinsically valuable products in the process according to the invention; the more intrinsically valuable saccharide structural units, on the other hand, are to be utilized separately as co-products.

Furthermore, the process according to the invention is to provide the products in such a form that they can be used as such in the foodstuffs industry, that is to say in particular without further working-up steps. Impurities, for example due to oligomeric secondary products or unreacted educts, are to be avoided where possible.

The object is achieved by the subject-matter of the patent claims.

A first aspect of the invention relates to an enzymatic process for the preparation of a product glucoside and of a co-product from an educt glucoside, wherein the process comprises the following steps:
(a) providing a first educt composition which comprises an educt glucoside phosphorylase, educt glucoside and phosphate;
(b) reacting at least a portion of the educt glucoside contained in the first educt composition with at least a portion of the phosphate contained in the first educt composition with catalysis by means of the educt glucoside phosphorylase, wherein there is obtained a first product composition which comprises co-product and glucose 1-phosphate;
(c) providing a second educt composition which comprises a product glucoside phosphorylase and at least a portion of the glucose 1-phosphate obtained in step (b), wherein a reactant for glucose 1-phosphate is also added, i.e. glucose; and
(d) reacting at least a portion of the glucose 1-phosphate contained in the second educt composition, with the added reactant for glucose 1-phosphate, i.e. glucose, with catalysis by means of the product glucoside phosphorylase, wherein there is obtained a second product composition which comprises the product glucoside and phosphate;
(e) (e') optionally, isolating at least a portion of the co-product; and/or
   (e") optionally, providing a third educt composition which comprises at least a portion of the co-product obtained in step (b) (or still contained in the second product composition obtained in step (d)) and a further enzyme capable of catalyzing a conversion of the co-product to a converted co-product;
   reacting at least a portion of the co-product contained in the third educt composition with catalysis by means of the further enzyme, wherein there is obtained a third product composition which comprises the converted co-product; and
   isolating at least a portion of the converted co-product; and
(f) optionally, isolating at least a portion of the product glucoside; and
(g) optionally, separating off the phosphate obtained in step (d) and optionally returning it to step (a).

It has been found, surprisingly, that the process according to the invention has a number of advantages as compared with conventional processes. For example, with the process according to the invention, the product glucoside, i.e. cellobiose, can be obtained in the form of a solid having defined properties (crystallinity, color, purity, water content, etc.), wherein the individual solid particles have a comparatively large grain size, are already colorless even without a decolorizing step, and are distinguished by a particularly high purity. Furthermore, the process according to the invention provides an improved yield, with regard to the educt glucoside used, for the product glucoside and for the co-product and thereby avoids the formation of undesirable secondary products, such as, for example, oligomers, which are formed in conventional processes when cellobiose is obtained from cellulose or starch. The content of unreacted glucose 1 -phosphate (educt) and optionally the content of unreacted reactant for glucose 1-phosphate is also low in the process according to the invention.

Further, it has been surprisingly found that the rate of reaction of the enzymatic process according to the invention is much faster than that of the processes according to the prior art, particularly than that of the process according to EP-A 423 768. The process according to the invention allows for a conversion of up to 70% of educt glucoside, i.e. sucrose, within 24 h, whereas according to EP-A 423 768 about 100 h are needed and within 24 h only about 50% conversion can be achieved.

Still further, it has been surprisingly found that reactions (b) and (d) can be conducted in a single aqueous phase containing essentially no organic solvents and essentially consisting of the educts, products and water. The phosphate acts as a buffer and no additional buffer is needed.

Yet further, it has been surprisingly found that the educt glucoside can be provided at a high concentration of at least 200 mM, preferably about 750 mM, and that the product glucoside can be obtained at a high concentration of at least 150 mM, preferably about 250 mM. As far as the product glucoside cellobiose is concerned, it has been surprisingly found that in spite of its solubility limit of 111 g/l, at 15°C does not precipitate from the second product composition.

Furthermore, it has been surprisingly found that the educt glucoside phosphorylase, the product glucoside phosphorylase, and additional enzymes, if any, may be employed repeatedly (i.e. recycled), for example by carrying out steps (b) and (d) in one or more membrane reactors, and that said enzymes do not need to be immobilized at solid supports that are located in separate reaction vessels (reactors). Further, there is no requirement for inactivating said enzymes after the reaction.

Moreover, it has been surprisingly found that steps (b) and (d), and optionally additional enzymatic conversions, if any, can be carried out in a single reactor (common reactor) such that the phosphate released in step (d) is simultaneously consumed in step (b), thereby keeping the overall phosphate concentration in said single reactor low.

The synthesis of the product glucoside from the educt glucoside takes place *via* the intermediate product glucose 1-phosphate. Glucose 1-phosphate and the co-product are first formed from the educt glucoside and from phosphate by means of an educt glucoside phosphorylase. Using a sucrose phosphorylase and sucrose as the educt glucoside it is possible, for example, to obtain the monosaccharide fructose as the co-product. It has been found, surprisingly, that the process according to the invention allows the co-product fructose to be obtained in an efficient manner. Fructose is a monosaccharide which is used in the production of soft drinks and confectionery and which is distinguished by a low glycaemic index despite its high energy content and good solubility in water.

By means of a second phosphorylase, glucose 1-phosphate is then transferred to a reactant for glucose 1-phosphate as co-substrate. The co-substrate can be, for example, a monosaccharide, disaccharide or another oligosaccharide. Using a cellobiose phosphorylase and glucose as co-substrate, the disaccharide cellobiose, for example, can be obtained as the product glucoside.

In the process according to the invention, phosphate is first consumed and then released again. The process according to the invention permits and preferably comprises the recovery of the phosphate, which constitutes a further, particular advantage of the process.

The process according to the invention can be carried out in one stage as a one-pot process and/or in two stages as a one- or two-pot process. In one stage in this context means that steps (b) and (d) of the process according to the invention take place simultaneously. Correspondingly, in two stages means that steps (b) and (d) of the process according to the invention take place temporally in succession. A person skilled in the art will appreciate that mixed forms with partial concurrence are also possible.

In a preferred embodiment, educt glucoside is metered in during the process as a fed-batch. This is preferred in particular when the educt glucoside phosphorylase and/or the product glucoside phosphorylase is inhibited by the presence of the educt glucoside in too high a concentration. By metering in the educt glucoside as a fed-batch, the concentration of the educt glucoside can be kept low and inhibition can thus be suppressed, while sufficient educt glucoside for the synthesis is nevertheless provided.

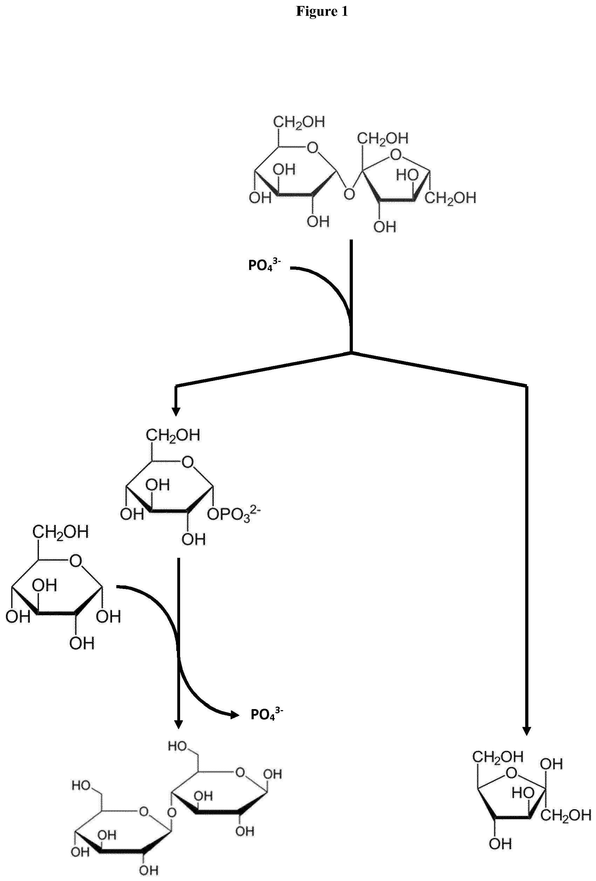

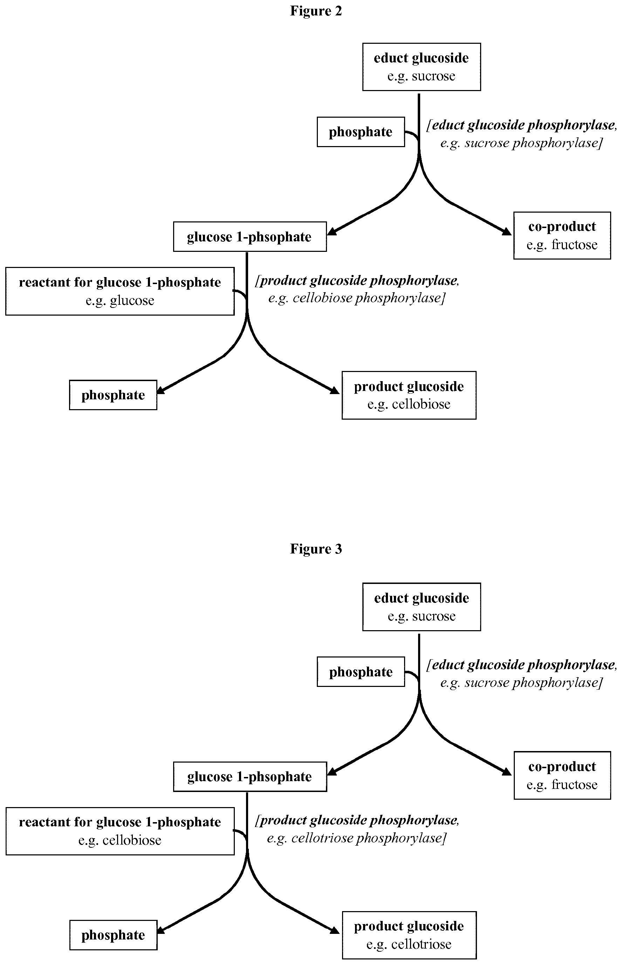

Figures 1 and 2 show schematically an inventive embodiment of the process according to the invention. Sucrose is thereby reacted as educt glucoside in the presence of phosphate, with catalysis by means of sucrose phosphorylase, to form glucose 1-phosphate and fructose as co-product. The glucose 1-phosphate is then reacted in the presence of glucose as reactant for glucose 1-phosphate, with catalysis by means of cellobiose phosphorylase, to form cellobiose with the release of phosphate. In this manner there are obtained from sucrose and glucose as starting materials cellobiose and fructose as products.

Figure 3 shows a variant of the embodiment that is shown in Figure 2 where the reactant for glucose 1-phosphate is not glucose but cellobiose and where the product glucoside phosphorylase is cellotriose phosphorylase to form cellotriose. In this manner there are obtained from sucrose and cellobiose as starting materials cellotriose and fructose as products.

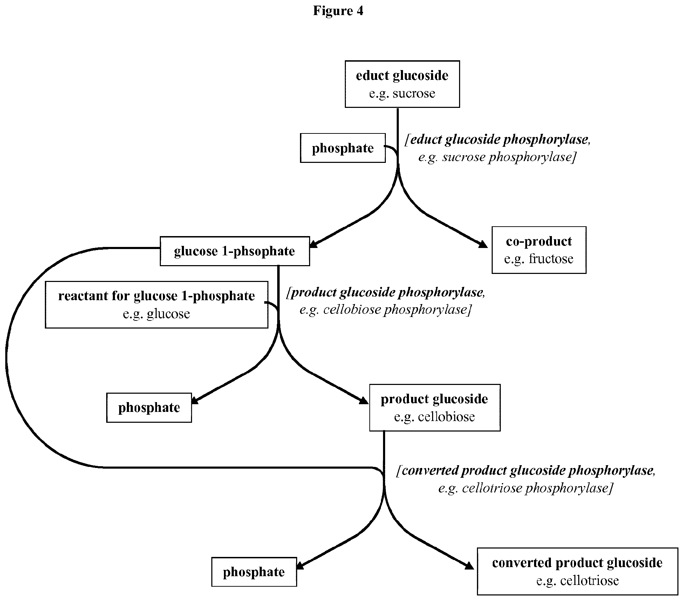

Figure 4 shows a variant of the embodiment that is shown in Figures 2 and 3 where the cellobiose that is obtained as product glucoside according to the embodiment according to Figure 2 is further reacted with glucose 1-phsophate. In this manner there are obtained from sucrose and glucose as starting materials cellotriose and fructose as products.

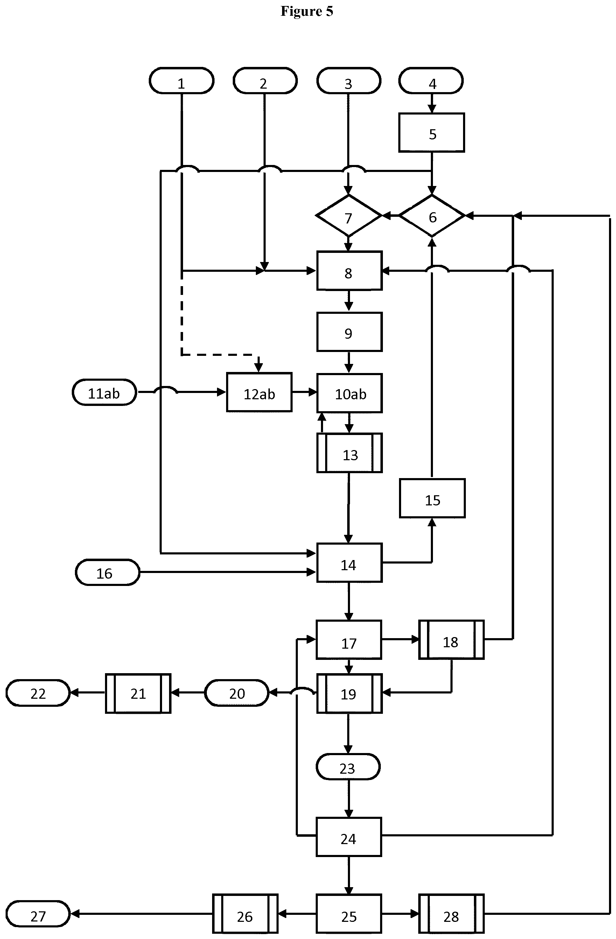

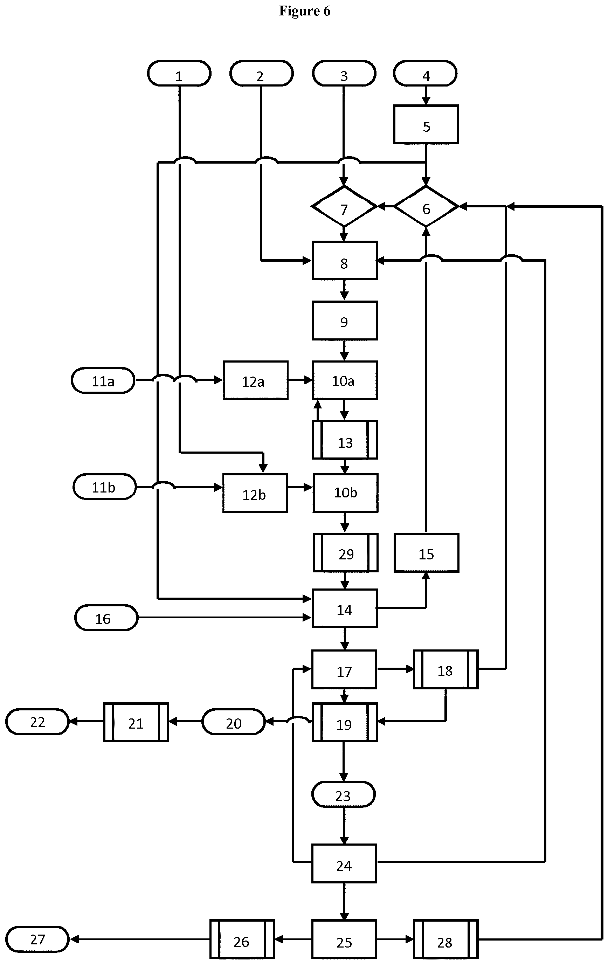

Figures 5 and 6 show schematically embodiments of the process as flow diagrams. Figure 5 shows the process according to the invention as a one-pot process, in which steps (b) and (d) are carried out in the same reactor. Figure 6 shows a reference process as a two-pot process, in which steps (b) and (d) are carried out spatially separately from one another in different reactors.

Steps (a) and (b) of the process according to the invention, on the one hand, and steps (c) and (d) of the process according to the invention are typically carried out temporally in succession. In a preferred embodiment of the process according to the invention, steps (c) and (d) are carried out temporally after steps (a) and (b), so that steps (a) to (d) are carried out in alphabetical order. It is, however, also possible according to the invention that one or more of the reaction steps is started before the previously started reaction step has ended completely. A person skilled in the art will appreciate that, in particular in the case of a preferably continuous procedure, the steps can all be carried out simultaneously.

Preferably, the process according to the invention is carried out as a continuous process. Preferably, steps (b) and (d) are carried out in a single aqueous phase which essentially contains no organic solvents.

Step (e) and optional step (f) of the process according to the invention can be carried out in any desired order or also simultaneously. Step (e) of the process according to the invention is preferably carried out after optional step (b) or after step (d), optional step (f) of the process according to the invention preferably being carried out after step (d).

Steps (a) to (e) and optional step (f) do not have to follow one another directly. Accordingly, it is possible according to the invention, and may even be preferred, for further, additional steps to be carried out between the individual steps, which additional steps differ in terms of measures from the measures according to steps (a) to (e) and optional step (f).

The process according to the invention is an enzymatic process, that is to say it takes place with enzymatic catalysis. The enzymes used are an educt glucoside phosphorylase in step (b) and a product glucoside phosphorylase in step (d). They are preferably two different enzymes.

The educt glucoside phosphorylase is a sucrose phosphorylase. Preferably, the sucrose phosphorylase is the sucrose phosphorylase of the wildtype of a bacterium belonging to the phylum of *Actinobacteria,* or a sucrose phosphorylase differing from said wildtype but comprising an amino acid sequence having at least 80% homology (identity) compared to the sucrose phosphorylase of the wildtype of a bacterium belonging to the phylum of *Actinobacteria.* Preferably, the bacterium belongs to the class of *Actinobacteria,* more preferably to the subclass of *Actinobacteridae,* still more preferably to the order of *Bifidobacteriales,* yet more preferably to the family of *Bifidobacteriaceae,* even more preferably to the genus of *Bifidobacterium,* and most preferably is *Bifidobacterium adolescentis.*

For the purpose of the specification, the homology is preferably calculated as identity using BLASTP (Stephen F. Altschul, Thomas L. Madden, Alejandro A. Schäffer, Jinghui Zhang, Zheng Zhang, Webb Miller, and David J. Lipman (1997) "Gapped BLAST and PSI-BLAST: a new generation of protein database search programs", Nucleic Acids Res. 25:3389-3402; Stephen F. Altschul, John C. Wootton, E. Michael Gertz, Richa Agarwala, Aleksandr Morgulis, Alejandro A. Schäffer, and Yi-Kuo Yu (2005) "Protein database searches using compositionally adjusted substitution matrices." FEBS J. 272:5101-5109), preferably using version BLASTP 2.2.29+ (http://blast.ncbi.nlm.nih.gov/Blast.cgi), preferably using the following settings:
- Field "Enter Query Sequence": Query subrange: none;
- Field "Choose Search Set": Database: non-redundant protein sequences (nr); optional parameters: none;
- Field "Program Selection": Algorithm: blastp (protein-protein BLAST);
- Algorithm parameters: Field "General parameters": Max target sequences: 100; Short queries: Automatically adjust parameters for short input sequences; Expect threshold: 10; Word size: 3; Max matches in a query range: 0;
- Algorithm parameters: Field "Scoring parameters": Matrix: BLOSUM62; Gap Costs: Existence: 11 Extension: 1; Compositional adjustments: Conditional compositional score matrix adjustment; and
- Algorithm parameters: Field "Filters and Masking": Filter: none; Mask: none.

SEQ ID NO:1 of the sequence listing is the amino acid sequence of the sucrose phosphorylase of the wildtype of *Bifidobacterium adolescentis.* SEQ ID NO:2 of the sequence listing is the amino acid sequence of the cellobiose phosphorylase of the wildtype of *Cellulomonas uda.* SEQ ID NO:3 of the sequence listing is the amino acid sequence of an engineered enzyme derived and thus differing from the wildtype cellobiose phosphorylase of *Cellulomonas uda.*

In preferred embodiments, when the sucrose phosphorylase differs from the wildtype but has at least 80% homology (identity) compared to the sucrose phosphorylase of the wildtype, preferably of *Bifidobacterium adolescentis* of SEQ ID NO:1, the identity is preferably at least 81%, or at least 82%, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99%.

The product glucoside phosphorylase is a cellobiose phosphorylase. Preferably, the cellobiose phosphorylase is the cellobiose phosphorylase of the wildtype of a bacterium belonging to the phylum of *Actinobacteria,* or a cellobiose phosphorylase differing from said wildtype but comprising an amino acid sequence having at least 80% identity compared to the cellobiose phosphorylase of the wildtype of a bacterium belonging to the phylum of *Actinobacteria.* Preferably, the bacterium belongs to the class of *Actinobacteria,* more preferably to the order of *Actinomycetales,* still more preferably to the suborder of *Micrococcineae,* yet more preferably to the family of *Cellulomonadaceae,* even more preferably to the genus of *Cellulomonas,* and most preferably is *Cellulomonas uda.*

In preferred embodiments, when the cellobiose phosphorylase differs from the wildtype but has at least 80% homology (identity) compared to the cellobiose phosphorylase of the wildtype, preferably of *Cellulomonas uda* of SEQ ID NO:2, the identity is preferably at least 81%, or at least 82%, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99%.

In preferred embodiments, when the cellobiose phosphorylase differs from the wildtype but has at least 80% identity compared to the cellobiose phosphorylase of the wildtype, preferably of the engineered enzyme derived from the cellobiose phosphorylase of *Cellulomonas uda* of SEQ ID NO:3, the identity is preferably at least 81%, or at least 82%, or at least 83%, or at least 84%, or at least 85%, or at least 86%, or at least 87%, or at least 88%, or at least 89%, or at least 90%, or at least 91%, or at least 92%, or at least 93%, or at least 94%, or at least 95%, or at least 96%, or at least 97%, or at least 98%, or at least 99%.

The process according to the invention is a preparation process. At least two reaction products are prepared, and an intermediate product is obtained intermediately. The first reaction product is the co-product obtained in step (b), which according to the invention is preferably not reacted further but is isolated as the product in step (e). The intermediate product is the glucose 1-phosphate obtained in step (b), of which at least a portion is reacted further in step (d). The second reaction product is the product glucoside obtained in step (d), which according to the invention is preferably not reacted further but is isolated as the product in optional step (f).

"Saccharides" within the meaning of the invention are carbohydrates, in particular any open-chained or cyclic, monomeric, oligomeric or polymeric polyhydroxycarbonyl compound. The term "saccharide" within the meaning of the invention includes in particular monosaccharides, disaccharides, trisaccharides and other oligosaccharides. According to the invention, the term "oligosaccharide" includes disaccharides, trisaccharides and longer-chained saccharides. If the D- or L-form is not mentioned expressly, then according to the invention it is preferably in each case the D-form.

"Glycoside" within the meaning of the invention is any saccharide which has a glycosidic bond at its anomeric carbon atom to a carbon, nitrogen, oxygen or sulfur atom of an aglycone or of another saccharide. If the glycosidic bond at the anomeric carbon atom joins one saccharide to the oxygen atom of another saccharide, the glycoside is itself an oligosaccharide. In this respect, the term glycoside within the meaning of the invention includes any oligosaccharides.

"Glucoside" within the meaning of the invention is a glycoside derived from glucose, in particular any glucose unit, in monomeric form or as a constituent of an oligosaccharide, which has a glycosidic bond at its anomeric carbon atom to a carbon, nitrogen, oxygen or sulfur atom of an aglycone or of another saccharide.

"Educt glucoside" within the meaning of the invention is the starting material (substrate) used in step (b) of the process according to the invention, the glycosidic bond of which is cleaved with enzymatic catalysis with phosphate (co-substrate) to form glucose 1-phosphate and the co-product. As the educt glucoside is sucrose, that is to say beta-D-fructofuranosyl-alpha-D-glucopyranoside, and the reaction in step (b) takes place with catalysis by means of sucrose phosphorylase, fructose is obtained as the co-product.

"Product glucoside" within the meaning of the invention is the product obtained in step (d) of the process according to the invention, the glycosidic bond of which is formed with enzymatic catalysis by reaction of glucose 1-phosphate (substrate) and optionally a reactant (co-substrate) with the release of phosphate. As the reactant is glucose and the reaction in step (d) takes place with catalysis by means of cellobiose phosphorylase, cellobiose, that is to say 4-O(heta-D-glucopyranosyl)-D-glucopyranose, is obtained as the product glucoside.

"Co-product" within the meaning of the invention is the product obtained in step (b) of the process according to the invention, which product is obtained together with glucose 1-phosphate by cleavage of the glycosidic bond of the educt glucoside with catalysis by means of the educt glucoside phosphorylase.

"Educt glucoside phosphorylase" within the meaning of the invention is the enzyme which is used in step (b) of the process according to the invention for the enzymatically catalyzed cleavage of a glycosidic bond of the educt glucoside with phosphate as co-substrate to form glucose 1-phosphate and the co-product. As the educt glucoside is sucrose, a sucrose phosphorylase, that is to say an enzyme which catalyzes this cleavage of the glycosidic bond, is used as the educt glucoside phosphorylase.

"Product glucoside phosphorylase" within the meaning of the invention is the enzyme which is used in step (d) of the process according to the invention for the enzymatically catalyzed formation of a glycosidic bond between glucose 1-phosphate and a further molecule of glucose 1-phosphate or optionally the reactant for glucose 1-phosphate. As the product glucoside is cellobiose, a cellobiose phosphorylase, that is to say an enzyme which catalyzes this formation of the glycosidic bond, is used as the product glucoside phosphorylase.

"Reaction" within the meaning of the invention includes any type of reaction procedure which is suitable for allowing the enzymatically catalyzed reaction to take place. In the simplest case, all the conditions which are required for the enzymatically catalyzed reaction are already established with the provision of the first or second educt composition. However, it is also possible according to the invention that, after the provision of the first or second educt composition, the reaction must be started by particular measures, for example by changing the temperature, the pH value, the addition of further components, such as, for example, salts.

"Glucose 1-phosphate" within the meaning of the invention is preferably alpha-D-glucose 1-phosphate.

"Reactant for glucose 1-phosphate" (reactant) within the meaning of the invention is any substance which, with catalysis by means of a suitable product glucoside phosphorylase, is capable of forming a glycosidic bond with glucose 1-phosphate with the release of phosphate. As the reactant for glucose 1-phosphate is glucose and a cellobiose phosphorylase is used as the product glucoside phosphorylase, cellobiose is formed as the product glucoside from glucose 1-phosphate and the reactant for glucose 1-phosphate with the release of phosphate.

"Phosphate" within the meaning of the invention is preferably orthophosphate and includes all partially or fully protonated forms of the PO₄³⁻ ion (H₃PO₄, H₂PO₄⁻, HPO₄²⁻, PO₄³⁻) as well as phosphate salts, in particular with cations selected from NH₄⁺, Na⁺, K⁺, Mg²⁺ and Ca²⁺.

"Isolation" within the meaning of the invention includes any suitable method for separating a component from a mixture of a plurality of components, the separated component preferably being separated off with the greatest possible purity. However, some impurities cannot be ruled out completely not least for thermodynamic reasons.

The term "essentially" in connection with the definition of the content of constituents (e.g. "essentially consisting of" or "containing essentially no") preferably means a content of other, i.e. not specified, constituents of not more than 2.5 wt.-%, more preferably not more than 1.0 wt.-%, most preferably not more than 0.5 wt.-%, and in particular not more than 0.1 wt.-%.

In step (a) of the process according to the invention, a first educt composition which comprises an educt glucoside phosphorylase, educt glucoside and phosphate is provided.

The first educt composition is preferably an aqueous solution in which the ion content, pH value, temperature, concentration of the educt glucoside phosphorylase, concentration of the educt glucoside, concentration of the phosphate, etc. are preferably so adjusted that the conditions are suitable for the reaction in step (b). Suitable conditions are known to a person skilled in the art and can be optimized by simple routine experiments.

The educt glucoside can in principle be any desired oligosaccharide which comprises at least one glycosidically bonded glucose unit. The educt glucoside is a disaccharide, i.e. sucrose.

Sucrose has excellent solubility in particular in the aqueous solutions that are of relevance for enzymatic processes, which solubility far exceeds that of starch and cellulose. In addition, phosphorylases which cleave sucrose with a selectivity close to 1 and thus without appreciable formation of secondary products are able to form glucose 1-phosphate and the co-product fructose with incorporation of an auxiliary substance.

The sucrose used as the educt glucoside can be of natural or chemical origin. The use of a solution comprising sucrose, such as raw juice, concentrated juice or molasses, is also possible.

The educt glucoside, i.e. sucrose, is provided in the first educt composition in a concentration of preferably from 0.10 mol/l to 5.00 mol/l, more preferably from 0.20 to 4.00 mol/l, yet more preferably from 0.40 to 3.00 mol/l, particularly preferably from 0.50 to 2.00 mol/l, most preferably from 0.60 to 1.50 mol/l and in particular from 0.70 to 1.00 mol/l, and is fed in that concentration to step (b) of the process according to the invention. Preferably, the concentration of the educt glucoside in the first educt composition provided in step (a) is at least 0.150 mol/l, more preferably at least 0.200 mol/l, still more preferably at least 0.300 mol/l, yet more preferably at least 0.400 mol/l, even more preferably at least 0.500 mol/l, most preferably at least 0.600 mol/l, and in particular at least 0.700 mol/l.

In a preferred embodiment, the educt glucoside is supplied continuously as a fed-batch, so that the concentration of the educt glucoside is not allowed to become too high.

The phosphate is preferably provided in step (a) of the process according to the invention in such a form that it is able to react in the cleavage, catalyzed by the educt glucoside phosphorylase, of the glycosidic bond of the educt glucoside and formation of glucose 1-phosphate in step (b) of the process according to the invention.

To that end, the phosphate is preferably in the form of orthophosphate and can be present in the form of a phosphate salt or in the form of phosphoric acid or a mixture thereof. The source for the phosphate is preferably phosphoric acid (H₃PO₄), disodium hydrogen phosphate (Na₂HPO₄), sodium dihydrogen phosphate (NaH₂PO₄), dipotassium hydrogen phosphate (K₂HPO₄) and/or potassium dihydrogen phosphate (KH₂PO₄).

The phosphate is provided in the first educt composition in a concentration of preferably from 0.00001 to 3.00 mol/l or 0.01 to 3.00 mol/l, more preferably 0.10 mol/l to 3.00 mol/l, still more preferably from 0.50 to 2.50 mol/l, yet more preferably from 0.60 to 2.00 mol/l, particularly preferably from 0.65 to 1.50 mol/l, most preferably from 0.70 to 1.20 mol/l and in particular 0.75 mol/l, and is fed in that concentration to step (b) of the process according to the invention. In a preferred embodiment, the phosphate is provided in the first educt composition in a concentration of preferably at most 3.00 mol/l, more preferably at most 1.00 mol/l, still more preferably at most 0.50 mol/l, yet more preferably at most 0.10 mol/l, particularly preferably at most 0.01 mol/l, most preferably at most 0.001 mol/l and in particular at most 0.00001 mol/l.

In principle, the co-product can likewise be any desired oligosaccharide which is shortened by at least one saccharide unit as compared with the educt glucoside by the formation of glucose 1-phosphate. The co-product is a monosaccharide, i.e. a ketohexose, namely fructose.

The educt glucoside phosphorylase is so chosen according to the invention that it catalyzes the cleavage of a glycosidic bond of the educt glucoside with phosphate as co-substrate with formation of glucose 1-phosphate and the co-product.

The educt glucoside phosphorylase is a disaccharide phosphorylase (EC 2.4.1).

The educt glucoside phosphorylase is a sucrose phosphorylase (EC 2.4.1.7, sucrose:phosphate alpha-D-glucosyltransferase), preferably a sucrose phosphorylase from *Bifidobacterium adolescentis, Bifidobacterium longum, Pelomonas saccharophila, Streptococcus mutans, Pseudobutyrivibrio ruminis, Shewanella putrefaciens* or *Leuconostoc mesenteroides.*

Inventive forms A¹ and reference forms A² and A³ for the educt glucoside, educt glucoside phosphorylase and the co-product obtained in each case are summarized in the table below:

| | Educt glucoside | Educt glucoside phosphorylase | Co-product |
|---|---|---|---|
| A¹ | Sucrose | Sucrose phosphorylase | Fructose |
| A² | Maltose | Maltose phosphorylase | Glucose |
| A³ | Lactose | Lactose phosphorylase | Galactose |

The synthesis activity of the educt glucoside phosphorylase is preferably in the range of from 0.1 to 25 U/ml, more preferably from 1 to 10 U/ml, yet more preferably from 2 to 8 U/ml, particularly preferably from 3 to 7 U/ml, most preferably from 4 to 6 U/ml and in particular 5 U/ml.

The synthesis activity of the educt glucoside phosphorylase is preferably in the range of from 0.1 to 25 U/mmol educt glucoside, more preferably from 0.25 to 20 U/mmol educt glucoside, yet more preferably from 0.5 to 15 U/mmol educt glucoside, particularly preferably from 0.75 to 10 U/mmol educt glucoside, most preferably from 1 to 8 U/mmol educt glucoside and in particular 5 U/mmol educt glucoside.

The educt glucoside phosphorylase can be used as a free enzyme in a stirred vessel reactor, a stirred vessel cascade or tubular reactor or as an immobilized enzyme in a stirred vessel, tubular or fixed bed reactor in a batch, repetitive batch or continuous procedure. In a preferred embodiment, the enzyme is separated from the product stream by ultrafiltration after the reaction.

In step (b) of the process according to the invention, at least a portion of the educt glucoside contained in the first educt composition is reacted with at least a portion of the phosphate contained in the first educt composition with catalysis by means of the educt glucoside phosphorylase, there being obtained a first product composition which comprises co-product and glucose 1-phosphate.

The enzymatic reaction with catalysis by means of the educt glucoside phosphorylase, i.e. sucrose phosphorylase, takes place in a temperature range of preferably from 15°C to 70°C, more preferably from 20°C to 60°C, yet more preferably from 25°C to 50°C, particularly preferably from 25°C to 40°C and most preferably at 30°C.

The enzymatic reaction with catalysis by means of the educt glucoside phosphorylase, i.e. sucrose phosphorylase, takes place at a pH value of preferably from pH 5 to pH 8, more preferably from pH 6 to pH 7 and particularly preferably at pH 6.3 or 6.5.

The concentration of the co-product and the concentration of the glucose 1-phosphate in the first product composition are not limited according to the invention and can be changed by a person skilled in the art optionally by selective, continuous or discontinuous separation of the co-product and of the glucose 1-phosphate.

In a preferred embodiment, the first product composition is fed to step (c) of the process according to the invention as an intermediate product, without further working up.

If steps (b) and (d) of the process according to a reference embodiment are carried out in separate reactors, the concentration of glucose 1-phosphate and co-product in the first product composition can be determined.

The concentration of glucose 1-phosphate in the first product composition is preferably from 0.10 mol/l to 2.00 mol/l, more preferably from 0.2 to 1.30 mol/l, yet more preferably from 0.4 to 1.1 mol/l, particularly preferably from 0.5 to 1.0 mol/l, most preferably from 0.6 to 0.9 mol/l and in particular from 0.7 to 0.8 mol/l. The glucose 1-phosphate is preferably fed in that concentration to step (d) of the process according to the invention.

The concentration of the co-product in the first product composition is preferably from 0.10 mol/l to 5.00 mol/l, more preferably from 0.2 to 4.00 mol/l, yet more preferably from 0.4 to 3.00 mol/l, particularly preferably from 0.5 to 2.00 mol/l, most preferably from 0.6 to 1.5 mol/l and in particular from 0.7 to 1.0 mol/l.

In a preferred embodiment, step (b) of the process according to the invention additionally comprises the subsidiary step
(b') separating off at least a portion of the resulting glucose 1-phosphate, preferably during the reaction of educt glucoside to form glucose 1-phosphate.

This embodiment is preferred in particular when steps (b) and (d) of the process are carried out in different reactors.

In a preferred embodiment, the glucose 1-phosphate is separated off by electrodialysis and/or crystallization. Preferably, the glucose 1-phosphate is separated off by electrodialysis and subsequently purified by crystallization.

As steps (b) and (d) of the process according to the invention are carried out in the same reactor, at least a portion of the resulting glucose 1-phosphate is preferably not separated off.

In a preferred embodiment of the process in which steps (b) and (d) of the process according to the invention are carried out in the same reactor, step (d) of the process according to the invention additionally comprises the subsidiary step
(d') separating off at least a portion of the resulting glucose 1-phosphate, preferably after the reaction of glucose 1-phosphate and optionally reactant to give product glucoside.

In step (c) of the process according to the invention, a second educt composition which comprises a product glucoside phosphorylase and at least a portion of the glucose 1-phosphate obtained in step (b) is provided.

The second educt composition is preferably provided in the form of the first product composition from step (b).

The second educt composition provided in step (c) of the process according to the invention additionally comprises a reactant for the glucose 1-phosphate, which is then reacted with the glucose 1-phosphate in step (d), with catalysis by means of the product glucoside phosphorylase, to form the product glucoside.

The second educt composition is preferably an aqueous solution in which the ion content, pH value, temperature, concentration of the product glucoside phosphorylase, concentration of the glucose 1-phosphate, optionally concentration of the reactant for glucose 1-phosphate, etc. are preferably so adjusted that the conditions are suitable for the reaction in step (d). Suitable conditions are known to a person skilled in the art and can be optimized by simple routine experiments.

In step (c) of the process according to the invention, the glucose 1-phosphate and/or the reactant for the glucose 1-phosphate are preferably added to the second educt composition independently of one another, together or separately, continuously or stepwise.

The reactant for the glucose 1-phosphate can in principle be any desired substance which has at least one nucleophilic functional group which is suitable for reacting with the glucose 1-phosphate in step (d) with enzymatic catalysis by means of the product phosphorylase. Suitable reactants are known to a person skilled in the art.

The reactant for the glucose 1-phosphate is different from the co-product.

In a reference embodiment, the reactant is an alcohol, particularly preferably glycerol, which then forms the aglycone of the product glucoside in step (d).

The reactant is a saccharide, i.e. an aldohexose, namely glucose. In a reference embodiment, the reactant is an aldohexose selected from the group consisting of allose, altrose, mannose, gulose, idose, galactose, and talose, in each case in D- and/or L-configuration. In another reference embodiment, the reactant is a ketohexose selected from the group consisting of allulose (psicose), fructose, sorbose, and tagatose, in each case in D- and/or L-configuration. In still another reference embodiment, the reactant is a nucleotide triphosphate, preferably selected from the group consisting of UTP, TTP, CTP, ATP, and GTP.

In another reference embodiment, the reactant is a disaccharide, preferably cellobiose. When glucose 1-phosphate is reacted with cellobiose in step (d) with enzymatic catalysis by means of cellodextrin phosphorylase (cellotriose phosphorylase) as the product phosphorylase, the resultant product glucoside is cellotriose.

The reactant is provided in the second educt composition in a concentration of preferably from 0.01 mol/l to 2.00 mol/l, more preferably from 0.10 to 1.30 mol/l, yet more preferably from 0.50 to 1.10 mol/l, particularly preferably from 0.65 to 0.90 mol/l, most preferably from 0.70 to 0.80 mol/l and in particular 0.75 mol/l, and is fed in that concentration to step (d) of the process according to the invention.

It has been found, surprisingly, that the preparation of cellobiose and fructose from sucrose with a co-substrate comprising phosphate, with catalysis by means of sucrose phosphorylase and cellobiose phosphorylase, also takes place directly and without the addition of a reactant for the glucose 1-phosphate as co-substrate, for example glucose. Two molecules of glucose 1-phosphate are then reacted to cellobiose. In a preferred embodiment of the process according to the invention, therefore, no further reactant for the glucose 1-phosphate is added in addition to the glucose 1-phosphate that is formed.

The product glucoside can in principle be any desired glucoside in dependence on the reactant for glucose 1-phosphate. The product glucoside is a disaccharide, i.e. cellobiose.

The product glucoside phosphorylase is so chosen according to the invention that it catalyzes the formation of a glycosidic bond between (i) the glucose 1-phosphate with glucose 1-phosphate or preferably (ii) the glucose 1-phosphate and optionally the reactant for glucose 1-phosphate with the release of phosphate as co-substrate with formation of the product glucoside.

The product glucoside phosphorylase is a disaccharide phosphorylase (EC 2.4.1).

The product glucoside phosphorylase is a cellobiose phosphorylase (EC 2.4.1.20, cellobiose:phosphate alpha-D-glucosyltransferase), preferably a cellobiose phosphorylase from *Cellvibrio gilvus, Ruminococcus albus, Ruminococcus flavefaciens, Thermogata maritima, Thermogata neapolitana, Clostridium thermocellum, Fomes annosus, Cellulomonas sp.* or *Cellulomonas uda.*

Inventive forms B¹ and B² and reference forms B³ to B³⁰ for reactants for the glucose 1-phosphate (D-glucose 1-phosphate), product glucoside phosphorylase and the product glucoside obtained in each case are summarized in the table below (the type of the released phosphate is also mentioned in the right column):

| | Reactant | Product glucoside phosphorylase | Product glucoside | Phosphate |
|---|---|---|---|---|
| B¹ | Glucose 1-phosphate | Cellobiose phosphorylase | Cellobiose | ortho Phosphate |
| B² | D-Glucose | Cellobiose phosphorylase | Cellobiose | ortho Phosphate |
| B³ | Glycerol | β-Glucosidase | Glycerol glucoside | ortho Phosphate |
| B⁴ | D-Allose | D-Glucosyl-D-alloside phosphorylase | D-Glucosyl-D-alloside | ortho Phosphate |
| B⁵ | L-Allose | D-Glucosyl-L-alloside phosphorylase | D-Glucosyl-L-alloside | ortho Phosphate |
| B⁶ | D-Altrose | D-Glucosyl-D-altroside phosphorylase | D-Glucosyl-D-altroside | ortho Phosphate |
| B⁷ | L-Altrose | D-Glucosyl-L-altroside phosphorylase | D-Glucosyl-L-altroside | ortho Phosphate |
| B⁸ | L-Glucose | D-Glucosyl-L-glucoside phosphorylase | D-Glucosyl-L-glucoside | ortho Phosphate |
| B⁹ | D-Mannose | D-Glucosyl-D-mannoside phosphorylase | D-Glucosyl-D-mannoside | ortho Phosphate |
| B¹⁰ | L-Mannose | D-Glucosyl-L-mannoside phosphorylase | D-Glucosyl-L-mannoside | ortho Phosphate |
| B¹¹ | D-Gulose | D-Glucosyl-D-guloside phosphorylase | D-Glucosyl-D-guloside | ortho Phosphate |
| B¹² | L-Gulose | D-Glucosyl-L-guloside phosphorylase | D-Glucosyl-L-guloside | ortho Phosphate |
| B¹³ | D-Idose | D-Glucosyl-D-idoside phosphorylase | D-Glucosyl-D-idoside | ortho Phosphate |
| B¹⁴ | L-Idose | D-Glucosyl-L-idoside phosphorylase | D-Glucosyl-L-idoside | ortho Phosphate |
| B¹⁵ | D-Galactose | D-Glucosyl-D-galactoside phosphorylase | D-Glucosyl-D-galactoside | ortho Phosphate |
| B¹⁶ | L-Galactose | D-Glucosyl-L-galactoside phosphorylase | D-Glucosyl-L-galactoside | ortho Phosphate |
| B¹⁷ | D-Talose | D-Glucosyl-D-taloside phosphorylase | D-Glucosyl-D-taloside | ortho Phosphate |
| B¹⁸ | L-Talose | D-Glucosyl-L-taloside phosphorylase | D-Glucosyl-L-taloside | ortho Phosphate |
| B¹⁹ | D-Allulose | D-Glucosyl-D-alluloside phosphorylase* | D-Glucosyl-D-alluloside | ortho Phosphate |
| B²⁰ | L-Allulose | D-Glucosyl-L-alluloside phosphorylase* | D-Glucosyl-L-alluloside | ortho Phosphate |
| B²¹ | L-Fructose | D-Glucosyl-L-fructoside phosphorylase* | D-Glucosyl-L-fructoside | ortho Phosphate |
| B²² | D-Sorbose | D-Glucosyl-D-sorboside phosphorylase* | D-Glucosyl-D-sorboside | ortho Phosphate |
| B²³ | L-Sorbose | D-Glucosyl-L-sorboside phosphorylase* | D-Glucosyl-L-sorboside | ortho Phosphate |
| B²⁴ | D-Tagatose | D-Glucosyl-D-tagatoside phosphorylase* | D-Glucosyl-D-tagatoside | ortho Phosphate |
| B²⁴ | L-Tagatose | D-Glucosyl-L-tagatoside phosphorylase* | D-Glucosyl-L-tagatoside | ortho Phosphate |
| B²⁶ | Uridine triphosphate (UTP) | UDP-Glucosephosphorylase (Glucose-1-phosphate uridyltransferase) | UDP-Glucose | Pyrophosphate |
| B²⁷ | Thymidine triphosphate (TTP) | TDP-Glucosephosphorylase (Glucose-1 -phosphate thymidyltransferase) | TDP-Glucose | Pyrophosphate |
| B²⁸ | Cytidine triphosphate | CDP-Glucosephosphorylase (Glucose-1-phosphate cytidyltransferase) | CDP-Glucose | Pyrophosphate |
| B²⁹ | Adenosine triphosphate (ATP) | ADP-Glucosephosphorylase (Glucose-1-phosphate adenylyltransferase) | ADP-Glucose | Pyrophosphate |
| B³⁰ | Guanosine triphosphate (GTP) | GDP-Glucosephosphorylase (Glucose-1-phosphate guanylyltransferase) | GDP-Glucose | Pyrophosphate |
| B³¹ | Cellobiose | Cellodextrin phosphorylase | Cellotriose | ortho Phosphate |
| B³² | (Glucose)n | β 1,2-oligoglucan phosphorylase | (β1,2-glucan)n | ortho Phosphate |

| | | | | |
|---|---|---|---|---|
| *It has been reported that sucrose phosphorylase from e.g. *Leuconostoc mesenteroides* has this activity (see K. Morimoto et al., J Biosc Bioeng 2015, 119(6), 652-656). | | | | |

The hydrolytic activity of the product glucoside phosphorylase is preferably in the range of from 0.1 to 25 U/ml, more preferably from 1 to 10 U/ml, yet more preferably from 2 to 8 U/ml, particularly preferably from 3 to 7 U/ml, most preferably from 4 to 6 U/ml and in particular 5 U/ml.

The hydrolytic activity of the product glucoside phosphorylase is preferably in the range of from 0.01 to 25 U/ml, more preferably from 0.05 to 10 U/ml, yet more preferably from 0.1 to 5 U/ml, particularly preferably from 0.5 to 3 U/ml, most preferably from 0.8 to 2 U/ml and in particular 1 U/ml.

The product glucoside phosphorylase can be used as a free enzyme in a stirred vessel reactor, a stirred vessel cascade or tubular reactor or as an immobilized enzyme in a stirred vessel, tubular or fixed bed reactor in a batch, repetitive batch or continuous procedure. In a preferred embodiment, the enzyme is separated from the product stream by ultrafiltration after the reaction.

In step (d) of the process according to the invention, at least a portion of the glucose 1-phosphate contained in the second educt composition is reacted with catalysis by means of the product glucoside phosphorylase, there being obtained a second product composition which comprises the product glucoside and phosphate.

The enzymatic reaction with catalysis by means of the product glucoside phosphorylase, i.e.cellobiose phosphorylase, takes place in a temperature range of preferably from 15°C to 70°C, more preferably from 20°C to 60°C, yet more preferably from 25°C to 55°C, particularly preferably from 30°C to 50°C and most preferably from 30°C to 40°C.

The enzymatic reaction with catalysis by means of the product glucoside phosphorylase, i.e. cellobiose phosphorylase, takes place at a pH value of preferably from pH 5 to pH 8, more preferably from pH 6 to pH 7 and particularly preferably at pH 6.3 or 6.5.

The concentration of the product glucoside and the concentration of the phosphate in the second product composition are not limited according to the invention and can be changed by a person skilled in the art optionally by selective, continuous or discontinuous separation of the product glucoside and of the phosphate.

The concentration of the phosphate in the second product composition is preferably from 0.01 mol/l to 2.00 mol/l, more preferably from 0.10 to 1.50 mol/l, yet more preferably from 0.40 to 1.10 mol/l, particularly preferably from 0.50 to 1.0 mol/l, most preferably from 0.60 to 0.90 mol/l and in particular from 0.70 to 0.80 mol/l.

The phosphate is provided in the second educt composition in a concentration of preferably from 0.00001 to 3.00 mol/l or 0.01 to 3.00 mol/l, more preferably 0.10 mol/l to 3.00 mol/l, still more preferably from 0.50 to 2.50 mol/l, yet more preferably from 0.60 to 2.00 mol/l, particularly preferably from 0.65 to 1.50 mol/l, most preferably from 0.70 to 1.20 mol/l and in particular 0.75 mol/l, and is fed in that concentration to step (b) of the process according to the invention. In a preferred embodiment, the phosphate is provided in the second educt composition in a concentration of preferably at most 3.00 mol/l, more preferably at most 1.00 mol/l, still more preferably at most 0.50 mol/l, yet more preferably at most 0.10 mol/l, particularly preferably at most 0.01 mol/l, most preferably at most 0.001 mol/l and in particular at most 0.00001 mol/l.

The concentration of the product glucoside, i.e. cellobiose, in the second product composition is preferably from 0.10 mol/l to 1.50 mol/l, more preferably from 0.15 to 1.30 mol/l, yet more preferably from 0.20 to 1.10 mol/l, particularly preferably from 0.25 to 1.00 mol/l, most preferably from 0.30 to 0.90 mol/l and in particular from 0.35 to 0.80 mol/l.

It has been surprisingly found that comparatively high concentrations of the product glucoside, i.e. cellobiose, in the second product composition can be achieved. Preferably, the concentration of the product glucoside, i.e. cellobiose, in the second product composition is at least 0.050 mol/l or at least 0.055 mol/l, more preferably at least 0.060 mol/l or at least 0.065 mol/l, still more preferably at least 0.070 mol/l or at least 0.075 mol/l, yet more preferably at least 0.080 mol/l or at least 0.085 mol/l, even more preferably at least 0.090 mol/l or at least 0.095 mol/l, most preferably at least 0.10 mol/l, at least 0.15 mol/l, at least 0.20 mol/l, or at least 0.30 mol/l, and in particular at least 0.40 mol/l, at least 0.50 mol/l, or at least 0.60 mol/l.

In a preferred embodiment, the co-product obtained in step (b) is not discharged until after step (d). The concentration of the co-product in the second product composition is then preferably from 0.10 mol/l to 2.00 mol/l, more preferably from 0.20 to 1.30 mol/l, yet more preferably from 0.40 to 1.10 mol/l, particularly preferably from 0.50 to 1.00 mol/l, most preferably from 0.60 to 0.90 mol/l and in particular from 0.70 to 0.80 mol/l.

The residual concentration of the educt glucoside in the second product composition is preferably not more than 0.30 mol/l, more preferably not more than 0.25 mol/l, yet more preferably not more than 0.20 mol/l, particularly preferably not more than 0.15 mol/l, most preferably not more than 0.1 mol/l and in particular not more than 0.05 mol/l. In a preferred embodiment, the educt glucoside can no longer be detected by analysis.

The residual concentration of the glucose 1-phosphate in the second product composition is preferably not more than 0.9 mol/l, more preferably not more than 0.8 mol/l, yet more preferably not more than 0.7 mol/l, particularly preferably not more than 0.6 mol/l, most preferably not more than 0.5 mol/l and in particular not more than 0.4 mol/l.

The residual concentration of the reactant for the glucose 1-phosphate, i.e. glucose, in the second product composition is preferably not more than 0.9 mol/l, more preferably not more than 0.8 mol/l, yet more preferably not more than 0.7 mol/l, particularly preferably not more than 0.6 mol/l, most preferably not more than 0.5 mol/l and in particular not more than 0.4 mol/l.

In a preferred embodiment of the process according to the invention, step (e) comprises step (e'), namely isolating at least a portion of the co-product.

In another preferred embodiment of the process according to the invention, step (e') comprises step (e"), namely
- providing a third educt composition which comprises at least a portion of the co-product obtained in step (b) and a further enzyme capable of catalyzing a conversion of the co-product to a converted co-product;
- reacting at least a portion of the co-product contained in the third educt composition with catalysis by means of the further enzyme, wherein there is obtained a third product composition which comprises the converted co-product; and
- isolating at least a portion of the converted co-product;
wherein step (e") preferably does not comprise the conversion of fructose to glucose and/or wherein the further enzyme is no glucose isomerase.

In a preferred embodiment, the converted co-product is not reacted in step (d) as reactant for the glucose 1-phosphate with at least a portion of the glucose 1-phosphate contained in the second educt composition with catalysis by means of the product glucoside phosphorylase, wherein there is obtained a second product composition which comprises the product glucoside and phosphate.

While it is principally possible that step (e) comprises both, step (e') as well as step (e"), it is preferred that step (e) comprises either step (e') but not step (e"), or step (e") but not step (e').

When process according to the invention comprises steps (a), (b), (c), (d) and (e'), it is a process for the preparation of a product glucoside and of a co-product from an educt glucoside.

When the process according to the invention comprises steps (a), (b), (c), (d) and (e"), however, the co-product is only obtained as an intermediate and further reacted to provide a converted co-product. Thus, this process may also be regarded as a process for the preparation of a product glucoside and of a converted co-product from an educt glucoside.

In alternative step (e') of the process according to the invention, at least a portion of the co-product is isolated. To that end, the co-product obtained in step (b) can be separated preferably from the first product composition or from the second product composition, preferably by membrane processes and/or chromatography processes, and in that manner can be purified and separated from the product glucoside. In a particularly preferred embodiment, the co-product is isolated after crystallization of the product glucoside, that is to say the product glucoside is preferably first crystallized from a mixture comprising product glucoside and co-product, and the co-product that remains in the supernatant is then isolated therefrom by means of the mentioned processes.

In a preferred embodiment, step (e') of the process according to the invention comprises separating off the co-product by means of chromatography or a membrane process, the membrane process preferably being a nanofiltration.

It has been found, surprisingly, that, by means of the process according to the invention, the co-product can be provided in such a form that it can be used as such, that is to say optionally even without further working-up steps, in the foodstuffs industry. As the co-product is fructose, it can be obtained in the form of pure fructose or in the form of fructose syrup. Preferably, a pure solution of the co-product, i.e. a fructose solution, is obtained according to the invention by chromatography. This solution is then preferably concentrated, for example by reverse osmosis or evaporation.

Sucrose is used as the educt glucoside and fructose is obtained as the co-product.

In a preferred embodiment, the separation of the co-product, i.e. fructose, from the reaction solution is carried out after the first enzymatic reaction (step (b)), preferably by a membrane process. In a preferred embodiment, a nanofiltration is used as the membrane process. In another preferred embodiment, the co-product, i.e. fructose, is separated by chromatography, preferably by means of batch chromatography and/or SMB (simulated moving bed chromatography).

In another preferred embodiment, the separation of the co-product, i.e. fructose, from the reaction solution is carried out after the second enzymatic reaction (step (d)), preferably by a membrane process. In a preferred embodiment, a nanofiltration is used as the membrane process. In another preferred embodiment, the co-product, i.e. fructose, is separated by chromatography, preferably by means of batch chromatography and/or SMB (simulated moving bed chromatography).

In a further preferred embodiment, the purity and yield of the co-product, i.e. fructose, are increased by the enzymatic isomerization of homologous sugars such as, for example, glucose to fructose or by separation of the co-products into pure monosaccharide fractions by a chromatographic process.

The chromatography can be carried out on non-ionic carriers. In a preferred embodiment, an ion-exchange resin (e.g. Lewatit® MDS 1368 in Ca, Na and K form in the particle size range from 290 to 320 µm or DOW Monoshere 99 in Ca, Na and K form in the particle size range from 290 to 320 µm or Purolite PCR642Ca in the particle size range 295 to 335 µm, or Purolite PCR642K in the particle size range 295 to 335 µm) is used as the solid phase in the chromatography.

The process according to the invention can employ various filtration techniques for working up, purifying and isolating the product composition, in particular nanofiltration, ultrafiltration and sterile filtration.

The separation of monosaccharides from disaccharides is preferably carried out by means of a nanofiltration, the pore size of the filters used for that purpose preferably being in the range of from 0.001 to 0.01 µm and the cut-off preferably being in the range of approximately from 0.1 to 2 kDa.

Retention or separation of the non-immobilized enzymes (educt glucoside phosphorylase and/or product glucoside phosphorylase) is preferably carried out by means of an ultrafiltration. Ultrafiltration membranes typically have a pore size range of approximately from 0.01 to 0.1 µm and the cut-off is preferably in the range of from 1 to 500 kDa, preferably at least 10 kDa. The ultrafiltration is preferably carried out at a temperature in the range of from 10 to 70°C, more preferably in the range of from 20 to 50°C and in particular at approximately 30°C.

Retention of microorganisms is preferably carried out by means of a sterile filtration, the pore size of the membrane preferably being about 0.2 µm.

Nanofiltration is a possible process technology for separating off secondary products and for purifying the products. Nanofiltration is a separation process based on membranes, in which molecules pass through the membrane in dependence on their size and the pore size of the membrane. Molecules of different sizes from process solutions can thus be separated from one another. US 6,406,546 discloses obtaining sucrose by nanofiltration from product streams which comprise monosaccharides and salts, which also include, for example, molasses. WO 2007/048880 relates to the separation of non-polar substances from polar substances by nanofiltration. The separation is thereby based on the effect that non-polar substances are retained at polar membranes to a greater extent than are polar substances. CN101376901A discloses the separation of monosaccharides, in particular fructose, from di- and fructooligosaccharide-containing solutions by nanofiltration. US 2009/0281305 discloses the isolation of cellobiose from a sugar mixture by ultrafiltration with the aid of a reverse osmosis membrane. The separation of monosaccharides from di- and oligo-saccharides is likewise known, the nanofiltration being used in cross-flow or dead-end operation (A.S. Grandison et al., The use of dead-end and cross-flow nanofiltration to purify prebiotic oligosaccharides from reaction mixtures in: Songklanakarin J. Sci. Technol., (2002), 24). US 5,869,297 discloses the purification of glucose by nanofiltration, the glucose having been produced by enzymatic starch saccharification.

The process according to the invention preferably comprises either a nanofiltration or a chromatography, that is to say they are preferred alternative purification steps. Chromatography can be preferred because it is sometimes more suitable for the purification of complex mixtures. Depending on the educt glucoside and reactant used, that is to say depending on the product glucoside and co-product obtained, nanofiltration membranes which permit effective purification may, however, also be available. In this case, a nanofiltration may be more economically advantageous than a chromatography and may therefore be preferred. A person skilled in the art can determine which of the two alternative processes may be best for which educts/products by means of simple routine experiments.

The use of chromatographic methods for purifying solutions which comprise different sugars is also known from the prior art. Liquid chromatography is used on an industrial scale for obtaining sugars, for example in obtaining sugars from molasses, separating glucose and fructose, and in the size grading of sugar molecules. The quality of the separation of sugar mixtures depends on the one hand on the physical and chemical properties of the carrier material, such as, for example, the structure, particle size, degree of crosslinking, capacity and ion type (e.g. Na⁺, Ca²⁺, K⁺), etc. and on the other hand on the interactions of the sugar molecules with the solvent. The separation of sugar molecules is conventionally carried out using ion-exchange resins with variable particle sizes between 290 and 370 µm and zeolites. The chromatography systems for separation can be configured for batch mode or as simulated moving bed systems (P.E. Barker et al., Continuous chromatographic separation of glucose-fructose mixtures using anion-exchange resins, Chromatographia 1984, 18, 567-574; M. Heper et al., Sodium, ammonium, calcium, and magnesium forms of zeolite Y for the adsorption of glucose and fructose from aqueous solutions, J. Colloid Interface Sci. 2007, 306, 11-15; S.M. Al Eid, Chromatographic separation of fructose from date syrup, Int. J. Food Sci. Nutr. 2006, 57, 83-96; and J.D. Sherman et al., Carbohydrate separations using zeolite molecular sieves, Stud. Surf. Sci. Catal. 1986, 28, 1025-1032).

A chromatographic separation of sugar mixtures which comprise cellobiose has not hitherto been described in the literature (US 2009/0281305 mentions the use of strongly acidic ion-exchange resins).

In the process according to the invention, a product stream, after it has optionally been pretreated by electrodialysis, preferably without prior further treatment, is preferably separated into its individual constituents by the use of a chromatography, water preferably being used as eluant. Pretreatment by electrodialysis is preferred when Ca resins are used, because calcium phosphate formation can otherwise occur.

In a preferred embodiment, step (e') of the process according to the invention is carried out temporally after step (d), that is to say the co-product is isolated only after at least a portion of the product glucoside has been obtained. Step (e') preferably comprises separating the co-product from a composition which comprises at least a portion of the product glucoside obtained in step (d). This composition can be the second product composition, so that step (e') of the process according to the invention is then carried out temporally immediately after step (d) of the process according to the invention. It is, however, also possible for further working-up steps to be carried out first, before step (e') is carried out.

In a preferred embodiment, step (e') of the process according to the invention yields the co-product with a purity of at least 95 wt.% DMB (on a dry matter basis), more preferably at least 97 wt.% DMB and in particular at least 99 wt.% DMB. In a preferred embodiment, step (e') of the process according to the invention yields the co-product with a purity of at least 50 wt.% DMB (on a dry matter basis), more preferably at least 75 wt.% DMB and in particular at least 90 wt.% DMB.

In a preferred embodiment, step (e') of the process according to the invention yields the co-product in the form of a syrup having a content of educt glucoside and/or product glucoside and/or reactant for the glucose 1-phosphate, independently of one another, of in each case not more than 5 wt.%, more preferably not more than 3 wt.% and in particular not more than 1 wt.%, in each case based on the total weight of the syrup. In a preferred embodiment, step (e') of the process according to the invention yields the co-product in the form of a syrup having a content of educt glucoside and/or product glucoside and/or reactant for the glucose 1-phosphate, independently of one another, of in each case not more than 50 wt.%, more preferably not more than 25 wt.% and in particular not more than 10 wt.%, in each case based on the total weight of the syrup.

In alternative step (e") of the process according to the invention, a third educt composition is provided which comprises at least a portion of the co-product obtained in step (b) and a further enzyme capable of catalyzing a conversion of the co-product to a converted co-product. Preferably, however, step (e") does not comprise the conversion of fructose to glucose.

In a preferred embodiment, the first product composition is fed to step (c) and the second product composition is fed to step (e") of the process according to the invention as an intermediate product, without further working up. Thus, the third educt composition is preferably provided in the form of the second product composition from step (d) which in turn is preferably provided in the form of the first product composition from step (b).

In a preferred embodiment, the third educt composition provided in step (e") of the process according to the invention does not comprise a reactant for the co-product. According to this embodiment, the co-product is preferably isomerized in step (e"), with catalysis by means of the further enzyme, to form the converted co-product. For example, as the co-product is fructose, the fructose is preferably epimerized to allulose as converted co-product with catalysis by means of D-tagatose-3-epimerase as the further enzyme, acting as D-fructose-3-epimerase.

In another preferred embodiment, the third educt composition provided in step (e") of the process according to the invention additionally comprises a reactant for the co-product, which is then reacted with the co-product in step (e"), with catalysis by means of the further enzyme, to form the converted co-product. For example, as the co-product is fructose, the reactant is preferably another fructose based glucoside.

The third educt composition is preferably an aqueous solution in which the ion content, pH value, temperature, concentration of the further enzyme, concentration of the co-product, optionally concentration of the reactant for the co-product, etc. are preferably so adjusted that the conditions are suitable for the reaction in step (e"). Suitable conditions are known to a person skilled in the art and can be optimized by simple routine experiments.

In step (e") of the process according to the invention, the co-product and/or optionally the reactant for the co-product are preferably added to the third educt composition independently of one another, together or separately, continuously or stepwise.

The converted co-product can in principle be any desired carbohydrate or derivative thereof.

The further enzyme is preferably an epimerase. As the co-product is fructose, the further enzyme is preferably a fructose epimerase.

Preferred forms C¹ to C³ for co-product, optional reactants for the co-product, further enzyme and the converted co-product obtained in each case are summarized in the table below:

| | Co-product | Reactant for co-product | Further enzyme | Converted co-product |
|---|---|---|---|---|
| C¹ | D-Fructose | - | D-Fructose-3-epimerase* | D-Allulose |
| C² | D-Fructose | Sucrose | Levansucrase, β-fructofuranosidase | 6-Kestose |
| C³ | D-Fructose | Sucrose | β-Fructofuranosidase | 1-Kestose |

| | | | | |
|---|---|---|---|---|
| *It has been reported that D-Tagatose-3-epimerase has this activity | | | | |

The further enzyme can be used as a free enzyme in a stirred vessel reactor, a stirred vessel cascade or tubular reactor or as an immobilized enzyme in a stirred vessel, tubular or fixed bed reactor in a batch, repetitive batch or continuous procedure. In a preferred embodiment, the enzyme is separated from the product stream by ultrafiltration after the reaction.

In step (e") of the process according to the invention, at least a portion of the co-product contained in the third educt composition is then reacted with catalysis by means of the further enzyme, there being obtained a third product composition which comprises a converted co-product.

In a preferred embodiment the further enzyme is added to the first educt composition or to the second educt composition or to the third educt composition.

The enzymatic reaction with catalysis by means of the further enzyme takes place in a temperature range of preferably from 15°C to 70°C, more preferably from 20°C to 60°C, yet more preferably from 25°C to 55°C, particularly preferably from 30°C to 50°C and most preferably from 30°C to 40°C.

The enzymatic reaction with catalysis by means of the further enzyme takes place at a pH value of preferably from pH 5 to pH 8, more preferably from pH 6 to pH 7 and particularly preferably at pH 6.5.

The concentration of the converted co-product in the third product composition are not limited according to the invention and can be changed by a person skilled in the art optionally by selective, continuous or discontinuous separation of the converted co-product.

The concentration of the converted co-product in the third product composition is preferably from 0.10 mol/l to 1.50 mol/l, more preferably from 0.15 to 1.30 mol/l, yet more preferably from 0.20 to 1.10 mol/l, particularly preferably from 0.25 to 1.00 mol/l, most preferably from 0.30 to 0.90 mol/l and in particular from 0.35 to 0.80 mol/l.

The residual concentration of the co-product in the third product composition is preferably not more than 0.9 mol/l, more preferably not more than 0.8 mol/l, yet more preferably not more than 0.7 mol/l, particularly preferably not more than 0.6 mol/l, most preferably not more than 0.5 mol/l and in particular not more than 0.4 mol/l.

In step (e") of the process according to the invention, at least a portion of the converted co-product is then isolated. Suitable steps for isolating the converted co-product are known to the skilled person and have been described above, e.g. with respect to the isolation of the co-product in step (e'). These steps analogously apply to the isolation of the converted co-product in step (e") and are thus not repeated hereinafter.

In a preferred embodiment, the converted co-product obtained in step (e") is used as reactant for the glucose 1-phosphate in step and (d) of the process according to the invention.

Preferably, when step (e) of the process according to the invention comprises step (e"), which in turn comprises sub-step of step (e") of isolating at least a portion of the converted co-product, said substep
e₁) comprises separating off the converted co-product by means of a chromatography or a membrane process; wherein the membrane process, where appropriate, is a nanofiltration; and/or
e₂) is carried out temporally after step (d); and/or
e₃) comprises separating the converted co-product from a composition which comprises at least a portion of the product glucoside obtained in step (d); and/or
e₄) yields the converted co-product with a purity of at least 0.1 wt.%, preferably at least 10%, more preferably at least 20%, still more preferably at least 30%, yet more preferably at least 40%, even more preferably at least 50%, most preferably at least 60% and in particular at least 70%, on a dry matter basis and/or yields the converted co-product in the form of a syrup having a content of educt glucoside and/or product glucoside of not more than 5 wt.%, based on the total weight of the syrup.

In optional step (f) of the process according to the invention, at least a portion of the product glucoside is isolated.

In the process according to the invention, the product glucoside is preferably purified, preferably from the second product composition, preferably by membrane and/or chromatographic processes, preferably in combination with precipitation, crystallization and/or drying processes. In this manner, monosaccharides (secondary products or unreacted educts) in particular are preferably separated from the product glucoside. The proportion of crystalline and amorphous product glucoside is preferably adjusted by the process step of drying, preferably spray drying.

It has been found, surprisingly, that it is possible by means of the process according to the invention to provide the product glucoside in such a form that it can be used as such, that is to say without further working-up steps, in the foodstuffs industry. As the product glucoside is cellobiose, it can be obtained in the form of a solid with defined crystallinity.

In a preferred embodiment, optional step (f) of the process according to the invention comprises separating off the product glucoside by means of a precipitation with an auxiliary substance, the auxiliary substance optionally comprising ethanol.

US 2009/0281305 discloses the crystallization of cellobiose from solutions. The cellobiose must thereby contain over 50% cellobiose, so that the content of monosaccharide (glucose) must be less than 50%. The purity of the cellobiose obtained by this process was 93%. A product that was as white as possible was obtained by an additional decolorizing step.

It has now been found, surprisingly, that cellobiose with a purity of over 95% can nevertheless be obtained according to the invention from a solution that comprises less than 50% cellobiose and thus more than 50% monosaccharides. In addition, it has been found, surprisingly, that the crystallization of cellobiose can also be carried out without previous decolorization.

The separation of the product glucoside preferably takes place by means of a precipitation step, preferably crystallization step with subsequent separation from the mother liquor by centrifugation. Preferably, precipitation, more preferably crystallization of the product glucoside is achieved by adding an anti solvent, preferably ethanol.

In a preferred embodiment, the crystals of the product glucoside, i.e. of cellobiose, are separated off by means of a centrifuge, whereby the crystals can optionally be washed with water in the centrifuge in order to reduce adhering discolorations. Such processes are known to a person skilled in the art.

In a preferred embodiment, optional step (f) of the process according to the invention comprises separating off the product glucoside by means of drying, whereby the product glucoside is obtained in the form of a solid. The product glucoside is thereby obtained at least in part in crystalline form. According to the invention, the drying preferably comprises spray drying, belt drying or drying by means of a spin flash dryer.

An advantage of spray drying is that the proportion of crystalline and amorphous product glucoside in the resulting spray-dried solid can thereby be varied.

In a preferred embodiment, optional step (f) of the process according to the invention comprises separating off the product glucoside by means of a membrane process, the membrane process preferably being a nanofiltration.

In a preferred embodiment, optional step (f) of the process according to the invention comprises hydrolyzing residual amounts of unreacted educt glucoside before the product glucoside is separated off.

In a preferred embodiment, optional step (f) of the process according to the invention comprises separating off the product glucoside by means of an ion exchanger on a solid carrier, whereby the product glucoside is separated from at least a portion of the phosphate.

In a particularly preferred embodiment, step (e) and optional step (f) of the process according to the invention are carried out together, so that the isolation (purification) of the product glucoside and the isolation (purification) of the co-product take place by a common measure, which according to the invention can comprise a plurality of subsidiary steps.

In a reference embodiment, the educt glucoside phosphorylase and/or the product glucoside phosphorylase are immobilized on a solid carrier and/or enclosed in a matrix independently of one another, together or separately. This facilitates the separation/retention of the two enzymes from the remaining components, for example isolation of the two enzymes from the first product composition or the second product composition.

According to the invention, the educt glucoside phosphorylase and/or the product glucoside phosphorylase, independently of one another, together or separately, are neither immobilized on a solid carrier nor enclosed in a matrix.

The use of immobilized enzymes in the process according to the reference embodiment allows the enzyme costs to be reduced. As a result of the immobilization, the enzymes can be recovered and used repeatedly. The immobilization of a sucrose phosphorylase from *Bifidobacterium adolescentis* in order to generate a thermostable system is known from the prior art (A. Cerdobbel et al., Increasing the thermostability of sucrose phosphorylase by multipoint covalent immobilization in: J. Biotechnol., (2010), 150). Using sucrose phosphorylase from *Bifidobacterium adolescentis* immobilized on Sepabeads EC-HFA it has been possible to achieve concentrations of up to 179 g of L-1 at a reaction temperature of up to 60°C (W.K. De et al., Operational stability of immobilized sucrose phosphorylase: Continuous production of α-glucose-1-phosphate at elevated temperatures in: Process Biochem. (Amsterdam, Neth.), (2011), 46). However, these works were carried out using particularly purified enzyme.

For reasons of cost it is preferred according to the reference embodiment to use immobilized enzymes which, prior to their immobilization, have not been purified by particular measures other than precipitation and filtration, but were isolated merely by PEI precipitation and filtration.

It has been found, surprisingly, that immobilization leads to consistent enzyme activity over 40 days. Surprisingly, up to 26% of the enzyme activity used is recovered. The activity of free enzyme which is retained by a membrane, on the other hand, falls continuously.

Immobilizing the enzymes from a non-purified enzyme solution, as is preferred according to the reference embodiment, allows the enzymes to be immobilized on a carrier, whereby the enzymes continue to be active, stable in the long term and selectively catalyze the desired reaction.

Alternatively, however, the two enzymes can also be isolated independently of one another by means of membrane processes, preferably by ultrafiltration. These processes are known to a person skilled in the art.

Membrane processes also allow the non-immobilized enzymes to be recovered and reused, as a result of which the enzyme costs can likewise be reduced. The enzymes are thereby separated from the product stream. An advantage of the membrane processes as compared with immobilization of the enzymes is that there are no costs for immobilizing the enzymes and a high loss of activity of the enzyme which is usually caused by immobilization is avoided. On the other hand, the long-term stability of non-immobilized enzymes is frequently lower than the long-term stability of immobilized enzymes.

In preferred embodiments of the process according to the invention, the educt glucoside phosphorylase, and/or the product glycoside phosphorylase, and/or the further enzyme are recycled. Thus, preferably the educt glucoside phosphorylase, and/or the product glycoside phosphorylase, and/or the further enzyme are not deactivated, e.g. by heat treatment.

Preferably, the process according to the invention is carried out in one membrane reactor. Preferably, the educt glucoside phosphorylase, and/or the product glycoside phosphorylase, and/or the further enzyme cannot pass the membrane of the membrane reactor. Preferably, the educt glucoside phosphorylase, and/or the product glycoside phosphorylase, and/or the further enzyme are not immobilized on carriers and are not provided in separate reactors.

It has been surprisingly found that performing steps (b) and (d) of the process according to the invention in one membrane reactor or in more than one membrane reactor according to a reference embodiment and employing the educt glucoside phosphorylase, the product glycoside phosphorylase, and the further enzyme, if any, in free, i.e. not immobilized form, a repeated use of the enzymes is possible. It has been surprisingly found that the retention of the enzymes by the membrane(s) of the membrane reactor(s) does not deteriorate enzymatic activity so that high recycling rates can be achieved. Immobilization and inactivation of enzymes can thus be avoided.

In preferred embodiments of the process according to the invention, the first educt composition and/or the second educt composition, independently of one another, is buffered by phosphate. Preferably, the first educt composition essentially consists of educt glucoside phosphorylase, educt glucoside, phosphate, and water. Preferably, the first product composition essentially consists of educt glucoside phosphorylase, unreacted educt glucoside, glucose 1-phosphate, co-product, unreacted phosphate, and water. Independently, the second educt composition preferably essentially consists of product glucoside phosphorylase, glucose 1-phosphate, optionally reactant for the glucose 1-phosphate, and water. Preferably, the second product composition preferably essentially consists of product glucoside phosphorylase, unreacted glucose 1-phosphate, unreacted reactant for the glucose 1-phosphate, product glucoside, and water. As steps (b) and (d) are conducted in a single reactor (common reactor), the overall reaction mixture preferably essentially consists of educt glucoside phosphorylase, product glucoside phosphorylase, educt glucoside, glucose 1-phosphate, reactant for the glucose 1-phosphate, product glucoside, co-product, phosphate, and water. Preferably, the first educt composition and/or the second educt composition, independently of one another, do not contain magnesium acetate, nor 4-morpholinepropanesulfonic acid (MOPS), nor imidazole hydrochloride.

In a particularly preferred embodiment, the process according to the invention comprises the additional step
(g) separating off the phosphate obtained in step (d) and preferably returning the phosphate which has been separated off to step (a).

One difficulty when developing an efficient enzymatic process for the preparation of product glucosides, in particular cellobiose, from educt glucosides, in particular from sucrose, is that phosphate is used as co-substrate in the enzymatic conversion of sucrose to glucose 1-phosphate in step (b) and is then released again as a secondary product in the enzymatic formation of the product glucoside in step (d). In the enzymatic formation of the product glucoside in step (d), product inhibition by phosphate occurs, while it is previously possible in the enzymatic conversion of educt glucoside to glucose 1-phosphate in step (b) to shift the equilibrium of the enzymatic reaction positively towards the intermediate glucose 1-phosphate by using a high phosphate concentration. During the reaction as a whole, control of the phosphate balance is therefore desirable.

One possibility according to the invention of controlling the phosphate balance is purposively to select educt glucoside phosphorylases that permit an advantageous reaction equilibrium for the formation of glucose 1-phosphate in step (b) even without a high phosphate concentration, and/or to select product glucoside phosphorylases that have an increased tolerance to phosphate in step (d).

Another possibility according to a reference embodiment of controlling the phosphate balance consists in separating phosphate from the first product composition after step (b) so that the second educt composition provided in step (c) has a lower content of phosphate. However, this is only possible in the case of a two-pot process, that is to say when step (b) and step (d) of the process according to the invention take place in different reactors which are spatially separate from one another. In a one-pot process according to the invention, the two enzymatic reactions take place in parallel in one reactor, and the phosphate is preferably separated from the product composition which comprises the product glucoside and preferably also the co-product.

In a preferred embodiment, step (g) of the process according to the invention comprises separating off the phosphate by means of a membrane process, in particular by electrodialysis, or by means of an ion exchanger on a solid carrier.

It has been found, surprisingly, that the electrodialysis is not only useful for removing phosphate, but additionally for removing colored impurities, i.e. for discoloring the product, particularly the second product composition. Such undesirable colored impurities are known from the prior art and are typically formed autocatalytically.

It has been found, surprisingly, that phosphate can be separated efficiently from the process by means of membrane processes. In a preferred embodiment of the inventive process, the phosphate is separated off before or after the conversion of the glucose 1-phosphate to the product glucoside.

In a preferred embodiment of the process, phosphate is separated from the process solution after the conversion of glucose 1-phosphate and the reactant for glucose 1-phosphate, i.e. glucose, to the product glucoside, i.e. cellobiose.

In an embodiment that is likewise preferred, the conversion of the educt glucoside, i.e. sucrose, to glucose 1-phosphate by means of a sucrose phosphorylase is carried out first, then phosphate is separated off by means of a membrane process, and then glucose 1-phosphate is converted to the product glucoside by means of the product glucoside phosphorylase. The newly formed phosphate is subsequently then separated off by a membrane process.

In a preferred embodiment of the inventive process, an electrodialysis is used as the membrane process.

In a further embodiment of the process, the two enzymatic reactions take place in parallel in one reactor (one-pot process), so that educt glucoside, i.e. sucrose, is converted by the reactant for glucose 1-phosphate, i.e. glucose, and phosphate directly to the product glucoside, i.e. cellobiose. Phosphate is then separated from a process solution which comprises product glucoside, i.e. cellobiose, the solution comprising phosphate which has been separated off preferably being recycled, that is to say returned to step (a).

Preferably, at least 50%, more preferably at least 60%, still more preferably at least 70%, yet more preferably at least 80%, even more preferably at least 90%, most preferably at least 90%, in particular at least 95%, of the phosphate that is separated off in step (g) is returned to step (a) as at least a portion of the phosphate that is contained in the first educt composition.

In a preferred embodiment, phosphate is obtained only after crystallization of the product glucoside, i.e. cellobiose, from the second product composition by electrodialysis. In this case, product glucoside, i.e. cellobiose, is first separated from the second product composition, which has not yet been freed of salts, by crystallization and centrifugation and then the discharge from the centrifuge is subsequently freed of salts by electrodialysis. This variant may have advantages with regard to the size of the system and also improves the efficiency of the electrodialysis.

Processes for electrodialysis are known from the prior art. US 4,787,790 discloses the separation of phosphate salts from a solution comprising glucose 1-phosphate in the presence of monosaccharides by electrodialysis. Glucose 1-phosphate is thereby produced from starch, for example potato starch. The phosphate is separated off after the reaction. JP 2005-041784 likewise discloses the purification of glucose 1-phosphate by the separation of glucose 1-phosphate together with phosphate from a reaction by monovalent non-ion-selective membranes, or electrodialysis, and isolation of glucose 1-phosphate from a phosphate-containing solution by electrodialysis.

In a preferred embodiment of the inventive process, electrodialysis as the membrane process is operated at voltages of from 1 V to 40 V, preferably from 10 to 30 V and particularly preferably from 15 to 25 V and most preferably at 20 V.

The electrodialysis is preferably carried out at a temperature in the range of from 10 to 50°C, more preferably from 20 to 40°C and in particular approximately 30°C.

In a preferred embodiment, the optional but preferred step (g) of the process according to the invention is carried out temporally after step (d), that is to say the phosphate is isolated only after at least a portion of the product glucoside has been obtained. Step (g) preferably comprises separating the phosphate from a composition which comprises at least a portion of the product glucoside obtained in step (d). This composition can be the second composition, so that step (g) of the process according to the invention is then carried out temporally immediately following step (d) of the process according to the invention. It is, however, also possible for further working-up steps first to be carried out before step (g) is carried out.

In a preferred embodiment of the process according to the invention, the reaction equilibrium is influenced in the direction of the product glucoside, for example by removing phosphate from the product composition obtained in step (d).

In another preferred embodiment of the process according to the invention, the reaction equilibrium is not influenced by removing phosphate from the product composition obtained in step (d). It has been found, surprisingly, that the educt glucoside can be reacted with a yield in the range of from 60 to 100% even under these conditions.

In another preferred embodiment of the process according to the invention, the yield of the product glucoside obtained in step (d) relative to the amount of the educt glucoside provided in step (a) is at least 60%, more preferably at least 65%, still more preferably at least 70%. Preferably, the conversion rate is such that after 24 hours the yield of at least 60% is achieved. Preferably, the conversion rate is such that after 48 hours the yield of the product glucoside of at least 70% is achieved.

As the process according to the invention is carried out as a one-pot process, the electrodialysis, equipped with heterogeneous ion-exchange membranes, allows the product solution comprising sugar to be freed of salts and the unreacted glucose 1-phosphate to be removed from the solution. By means of a further electrodialysis, equipped with selective homogeneous ion-exchange membranes, for example based on styrene-divinylbenzene copolymers, the glucose 1-phosphate can be separated from the other salts. In this manner, in the one-pot process, the product solution comprising sugar is freed of salts after the reaction preferably by the use of an electrodialysis, preferably equipped with heterogeneous ion-exchange membranes, and the unreacted glucose 1-phosphate is removed from the solution. The concentrate of the electrodialysis is preferably not purified further and is used again for a new enzyme-catalyzed process.

Where the process according to a reference embodiment is carried out as a two-pot process, the electrodialysis, preferably equipped with selective homogeneous ion-exchange membranes, allows the excess salts of the buffer to be separated off after the first enzyme-catalyzed synthesis (step (b)). The glucose 1-phosphate preferably remains in the first product composition and is fed to the second enzyme-catalyzed synthesis (step (d)). The product of the second enzyme-catalyzed synthesis, that is to say the product glucoside in the second product composition, is preferably freed of salts after the reaction by the use of an electrodialysis, preferably equipped with heterogeneous ion-exchange membranes, and the unreacted glucose 1-phosphate is removed from the solution. The concentrate of the electrodialysis is then preferably likewise not purified further and is used again for a new enzyme-catalyzed process.

In a further preferred procedure, the electrodialysis is carried out after the product glucoside has been separated off by a crystallization process.

Preferably no glucose isomerase is used in the process according to the invention, particularly preferably no further enzymes are used apart from the educt glucoside phosphorylase and the product glucoside phosphorylase. In another preferred embodiment, a further enzyme is used in step (e") in order to convert to co-product into a converted co-product.

In order to increase the product yield it can, however, also be preferred to convert unreacted reactant for glucose 1-phosphate, i.e. glucose, into co-product, i.e. fructose, with enzymatic catalysis, provided this is technically possible and economically expedient. An enzyme which catalyzes the isomerization of glucose to fructose is glucose isomerase.

In a further preferred embodiment, an ethanol precipitation or a crystallization is used to obtain the product glucoside.

The process according to the invention can be carried out as a continuous, batch or repetitive batch process.

In a particularly preferred embodiment, the process according to the invention is operated continuously, preferably *via* a membrane reactor system, wherein the enzymes are retained by the membrane, wherein the product glucoside and the co-product are preferably removed continuously from the reactor, and wherein fresh educt glucoside and optionally fresh reactant for the glucose 1-phosphate are preferably added simultaneously or in a temporally offset manner.

In the process according to the invention, steps (b) and (d) are carried out in a common reactor (one-pot process). In this process variant, the first product composition obtained in step (b) is then provided for the second educt composition directly and without further working up, it being possible, however, for further components for the reaction according to step (d) to be added to the second educt composition. The first educt composition, the first product composition and/or the second educt composition coincide, and the single educt composition preferably contains both enzymes simultaneously from the start.

The one-pot process according to the invention can be carried out as a continuous, batch or repetitive batch process.

In a preferred embodiment of the one-pot process according to the invention, all the reactants, enzymes and conditions are already present and adjusted in the first educt composition provided in step (a) in such a manner that steps (b) to (d) of the process according to the invention proceed spontaneously, optionally until the reaction is complete. The provision of the second educt composition in step (c) then takes place by the reaction in step (b), whereby the first product composition serves as the second educt composition. In the case of a continuous procedure, educt glucoside, phosphate and optionally reactant for glucose 1-phosphate are metered in continuously, so that the reaction does not come to a standstill; to that end, product glucoside, co-product and phosphate are then discharged continuously.

In another preferred embodiment of the one-pot process according to the invention, reactants and/or enzymes that are required for the reaction in step (d) are missing from the educt composition provided in step (a). In the case of this procedure, after the provision of the first educt composition according to step (a), only the reaction according to step (b) initially takes place in the reactor, whereby the first product composition is formed in the reactor. However, the glucose 1-phosphate thereby formed does not spontaneously react further, because reactants and/or enzymes (product glucoside phosphorylase) that are required for the reaction in step (d) are missing. At a time which can in principle be chosen freely, for example when the reaction according to step (b) is complete, the missing reactants and/or enzymes that are required for the reaction in step (d) can be added to the first product composition contained in the reactor in order to provide the second educt composition according to step (c). The reaction according to step (d) can thus follow in the reactor.

In a reference embodiment of the process according to the invention, steps (b) and (d) are carried out in separate reactors (two-pot process). The two-pot process according to the invention can be carried out as a continuous, batch or repetitive batch process.

In this procedure, the two enzymes are preferably used spatially separately in the two reactors, so that step (b) proceeds in the first reactor and step (d) proceeds in the second reactor. Therefore, the reactant for glucose 1-phosphate and the product glucoside phosphorylase are preferably also added only when the first product composition has left the first reactor.

In preferred embodiments, step (e) and optional step (f) are embedded as constituents in a multistage working-up process, the ultimate aim of which is to isolate product glucoside and co-product in pure form and separate off the remaining components (secondary products, unreacted educts) and optionally return them to the process in a loop.

In a preferred embodiment, glucose 1-phosphate is discharged from the process according to the invention as a further co-product.

In a preferred embodiment, the purification of the process solution in order to obtain the product glucoside, i.e. cellobiose, is carried out *via* membrane processes and isolation in solid form by precipitation. In this case, the process according to the invention comprises a total of four sections, each of which can optionally comprise a plurality of steps of the process according to the invention:
(i) In the first section, the synthesis of the glucose 1-phosphate from educt glucoside, i.e. sucrose, and phosphate takes place with catalysis by means of an educt glucoside phosphorylase (steps (a) and (b)).
(ii) In the second section, the reaction of the glucose 1-phosphate with a reactant for glucose 1-phosphate, i.e. glucose, takes place with catalysis by means of a product glucoside phosphorylase, whereby the product glucoside is obtained, i.e. cellobiose (steps (c) and (d)).
(iii) In the third section, the separation of monosaccharides (co-product, i.e. fructose and unreacted reactant for glucose 1-phosphate, i.e. glucose) takes place (step (e)). Isomerization of the unreacted reactant, i.e. glucose, to co-product, i.e. fructose, by means of a suitable enzyme, for example a glucose isomerase, preferably takes place.
(iv) In the fourth section, purification of the product glucoside, i.e. cellobiose, takes place by precipitation or chromatography and/or crystallization and subsequent drying (optional step (f)).

In a preferred embodiment, following step (d), the concentrated solution is subjected to an electrodialysis at a voltage of from 2 V to 40 V, preferably at from 10 to 30 V, a stream comprising phosphate salt and glucose 1-phosphate being separated from the process solution. In a preferred embodiment, the remaining process solution is returned to the process again, preferably for blending of the educt glucoside. In a two-pot process, glucose 1-phosphate can be separated from the process solution comprising phosphate and glucose 1-phosphate preferably by means of an electrodialysis, and the material stream comprising phosphate can be used preferably for blending of the educt glucoside and the material stream comprising glucose 1-phsophate can be fed to the second enzymatic reaction (step (d), catalysis by product glucoside phosphorylase).

In a preferred embodiment, the process solution, which has been freed of phosphates, contains as main constituents unreacted reactant for glucose 1-phosphate, i.e. glucose, co-product, i.e. fructose, product glucoside, i.e. cellobiose, and as secondary constituent unreacted educt glucoside, i.e. sucrose, is filtered by means of a nanofiltration or separated in a chromatographic separation process, wherein monosaccharides such as glucose and fructose are separated from the process solution as a co-product stream.

In an embodiment that is likewise preferred, this co-product stream is reacted with an enzyme, preferably with glucose isomerase, whereby the fructose content is increased.

In a further preferred embodiment, this co-product stream is separated by means of a separating process, such as, for example, a chromatography, into three (or optionally also only into two) streams, wherein a first stream comprises unreacted reactant for glucose 1-phosphate, i.e. glucose, a second stream comprises the co-product, i.e. fructose, and a third stream comprises the product glucoside, i.e. cellobiose. The first stream is fed in a one-pot process preferably for blending of the educt glucoside or in a reference embodiment in a two-pot process preferably to the second enzymatic reaction (step (d), catalysis by product glucoside phosphorylase).

In a preferred embodiment, the co-product stream is concentrated to a concentration of from 40 wt.% DMB to 90 wt.% DMB, more preferably from 60 wt.% DMB to 90 wt.% DMB, yet more preferably from 65 wt.% DMB to 85 wt.% DMB, and is obtained in the form of a marketable co-product, preferably in the form of fructose syrup. The content of co-product, i.e. fructose, on a dry matter (DM) basis, is preferably at least 90 wt.%, more preferably at least 95 wt.%, particularly preferably at least 99 wt.%, in each case based on the total weight of the dry matter (purity). To that end, it can be preferred to feed the co-product stream to a device for decolorization, so that the co-product is largely colorless.

In a further preferred embodiment, the co-product stream is fed to a crystallization/spray drying, in order to achieve a crystalline product.

In another preferred embodiment, a product-containing fraction obtained by nanofiltration is separated in a separation process, such as a chromatography, into a product-containing fraction and a fraction comprising auxiliary substance, for example H₃PO₄, NaH₂PO₄, Na₂HPO₄, co-product, i.e. fructose, educt glucoside, i.e. sucrose, reactant for glucose 1-phosphate, i.e. glucose, secondary product, for example glucose 6-phosphate, and intermediate product, for example glucose 1-phosphate. The product-containing fraction is preferably dried in a spray dryer to give a solid. The other fractions comprising educts, intermediate product, auxiliary substance, secondary product and co-product are preferably combined, concentrated by optionally multistage evaporation, and can be discharged from the process as "product molasses".

In an alternative embodiment of the process according to the invention, step (f) does not necessarily involve the step of isolating at least a portion of the product glucoside (step (f)), but instead the step (f"
) providing a fourth educt composition which comprises at least a portion of the product glucoside obtained in step (d) and a further enzyme capable of catalyzing a conversion of the product glucoside to a converted product glucoside;
reacting at least a portion of the product glucoside contained in the fourth educt composition with catalysis by means of the further enzyme, wherein there is obtained a fourth product composition which comprises the converted product glucoside; and
isolating at least a portion of the converted product glucoside.

All preferred embodiments that have been described above in connection with step (f), i.e. the isolation of at least a portion of the product glucoside, also analogously apply to alternative step (f"), particularly to its sub-step of isolating at least a portion of the converted product glucoside, and thus are not repeated hereinafter.

A particularly preferred embodiment of the process according to the invention relates to a process for the preparation of a product glucoside and of a co-product from an educt glucoside, wherein the process comprises the following steps:
(a) providing a first educt composition which comprises an educt glucoside phosphorylase, educt glucoside and phosphate; wherein the concentration of the educt glucoside in the first educt composition is at least 0.150 mol/l; preferably at least 0.200 mol/l; more preferably at least 0.500 mol/l;
(b) reacting at least a portion of the educt glucoside contained in the first educt composition with at least a portion of the phosphate contained in the first educt composition with catalysis by means of the educt glucoside phosphorylase, wherein there is obtained a first product composition which comprises co-product and glucose 1-phosphate;
(c) providing a second educt composition which comprises a product glucoside phosphorylase, at least a portion of the glucose 1-phosphate obtained in step (b), and a reactant for glucose 1-phosphate; and
(d) reacting at least a portion of the glucose 1-phosphate contained in the second educt composition, optionally with the reactant for glucose 1-phosphate with catalysis by means of the product glucoside phosphorylase, wherein there is obtained a second product composition which comprises the product glucoside and phosphate; wherein the concentration of the product glucoside in the second product composition is at least 0.075 mol/l; preferably at least 0.150 mol/l; more preferably at least 0.200 mol/l;
(e') isolating at least a portion of the co-product; wherein preferably the co-product is separated off by means of a chromatography or a membrane process; wherein preferably the membrane process is a nanofiltration; wherein preferably the co-product is separated from a composition which comprises at least a portion of the product glucoside obtained in step (d);
(f) isolating at least a portion of the product glucoside; wherein preferably the product glucoside is separated off by means of
   - a membrane process; wherein preferably the membrane process is a nanofiltration; and/or
   - an ion exchanger on a solid carrier, whereby the product glucoside is separated from at least a portion of the phosphate; and/or
   - a crystallization step with subsequent separation of the crystals from the mother liquor by centrifugation; wherein preferably an anti solvent is added; wherein the anti solvent is preferably ethanol; and
(g) separating off the phosphate obtained in step (d), and preferably returning it to step (a); wherein the phosphate is separated off by means of a membrane process or an ion exchanger on a solid carrier; wherein the membrane process involves an electrodialysis; wherein preferably at least 50% of the phosphate that is separated off in step (g) is returned to step (a) as at least a portion of the phosphate that is contained in the first educt composition;
   wherein
   - the educt glucoside phosphorylase is a sucrose phosphorylase; preferably the sucrose phosphorylase of the wildtype of *Bifidobacterium adolescentis,* or a sucrose phosphorylase differing from said wildtype but comprising an amino acid sequence having at least 80%, preferably at least 90% identity compared to the sucrose phosphorylase of the wildtype of *Bifidobacterium adolescentis*; wherein preferably the educt glucoside phosphorylase is neither immobilized on a solid carrier nor enclosed in a matrix;
   - the product glucoside phosphorylase is a cellobiose phosphorylase; preferably the cellobiose phosphorylase of the wildtype of *Cellulomonas uda,* or a cellobiose phosphorylase differing from said wildtype but comprising an amino acid sequence having at least 80%, preferably at least 90% identity compared to the cellobiose phosphorylase of the wildtype of *Cellulomonas uda*; wherein preferably the product glucoside phosphorylase is neither immobilized on a solid carrier nor enclosed in a matrix;
   - the educt glucoside is sucrose,
   - the reactant for the glucose 1-phosphate is glucose,
   - the product glucoside is cellobiose, and
   - the co-product is fructose;
wherein steps (b) and (d) are carried out at least partially simultaneously in a common reactor; wherein preferably the common reactor is a membrane reactor; wherein preferably the educt glucoside phosphorylase and the product glycoside phosphorylase cannot pass the membrane of the membrane reactor.

Particularly preferred embodiments of the process according to the invention will be described in greater detail below by means of Figures 5 and 6. A person skilled in the art will appreciate that not all the devices depicted in Figures 5 and 6 must necessarily be provided in order to carry out the process according to the invention, and that further devices which are not depicted may also be provided.

Figure 5 shows the process according to the invention as a one-pot process in which steps (b) and (d) are carried out in the same reactor.

The reactant for the glucose 1-phosphate, educt glucoside, phosphate and water are preferably provided from a raw material store for the reactant for glucose 1-phosphate (1), from a raw material store for the educt glucoside (2), from a raw material store for the phosphate (3) and from a water tank (4). The water can optionally be treated by means of a device for softening and treatment (5), for example by ion exchange and/or reverse osmosis. The reactant for glucose 1-phosphate, the educt glucoside, the phosphate and the water are fed *via* separate lines preferably to the mixing container (8), whereby the water can be metered in optionally by means of a device for adjusting the concentration of the water (6) and the phosphate can be metered in optionally by means of a device for adjusting the concentration of the phosphate (7). This mixture, which preferably does not yet contain enzyme, can subsequently be prepared for the reaction preferably by means of a device for sterile filtration, heating, UV disinfection (9), or a combination of those processes. The two enzymes are preferably provided from a raw material store for the educt glucoside phosphorylase and the product glucoside phosphorylase (11ab). The mixture of the enzymes is preferably first pretreated by means of a device for sterile filtration of the educt glucoside phosphorylase and of the product glucoside phosphorylase (12ab) before it is fed *via* corresponding lines to the one-pot reactor, that is to say the reactor for reaction with catalysis by means of the educt glucoside phosphorylase and for reaction with catalysis by means of the product glucoside phosphorylase (10ab), where it is combined with the sterile-filtered and/or heated and/or UV-disinfected mixture of the reactants (steps (a) and (c)). In a preferred variant of this procedure (indicated by the broken line), the reactant for glucose 1-phosphate is not fed to the mixing container (8) but to the device for sterile filtration of the educt glucoside phosphorylase and of the product glucoside phosphorylase (12ab) and is therein combined with the enzymes (feed-batch option).

The two reactions according to steps (b) and (d) then take place simultaneously in the one-pot reactor (10ab).

The isolation according to step (e) and optional step (f) then takes place preferably *via* a plurality of stages. The product composition from the one-pot reactor (10ab) is preferably first pretreated by a device for ultrafiltration (13), constituents thereby separated off, in particular the enzymes, preferably being returned to the reactor. The mixture preferably so pretreated is then preferably fed to a device for electrodialysis (14). The electrodialysis (14) is preferably supplied with liquid KNO₃ as electrode rinse solution from a raw material store for potassium nitrate (16). The aqueous phase from the electrodialysis (14), which has a low product and phosphate content and is optionally enriched with unreacted glucose 1-phosphate, is preferably treated in a device for decolorization (15) and finally returned to the device for adjusting the concentration of the water (6) and to the device for adjusting the concentration of the phosphate (7). Alternatively, a further electrodialysis (not depicted) can follow, in order to obtain glucose 1-phosphate. The product-rich phase from the electrodialysis (14) is preferably treated in a device for crystallization (17) of the product glucoside, from which the vapor phase is preferably passed into a device for condensation (18). In a centrifuge/filter press (19), the product glucoside which has crystallized out is preferably separated from the liquid phase with >98% purity (20) and preferably dried in a device for drying (21) to give pure product glucoside (22). The mother liquor from the centrifuge/filter press (19), that is to say the solution comprising product glucoside, co-product and reactant for glucose 1-phosphate (23), is preferably separated in a device for SMB (simulated moving bed chromatography) (24), the chromatography preferably producing three streams: a stream comprising the co-product, a stream comprising unreacted reactant for glucose 1-phosphate, and a stream comprising product glucoside. The stream comprising the co-product from the device for SMB (24) is preferably treated in a further device for crystallization (25), and the co-product that has crystallized out is preferably dried in a device for drying (26), whereby pure, dried co-product (27) is obtained. Alternatively, the stream comprising the co-product is concentrated by an evaporator and decolorized, whereby a liquid concentrated co-product solution is obtained (not depicted). The vapor phase from the further device for crystallization (25) or of the evaporator for concentration is preferably condensed in a further device for condensation (28) and preferably likewise returned to the device for adjusting the concentration of the water (6) and to the device for adjusting the concentration of the phosphate (7). The stream comprising the unreacted reactant for glucose 1-phosphate from the device for SMB (24) is preferably returned to the mixing container (8). The stream comprising the product glucoside from the device for SMB (24) is preferably returned to the device for crystallization (17).

Figure 6 shows a reference process as a two-pot process, in which steps (b) and (d) are carried out spatially separately from one another in different reactors. In contrast to the process according to Figure 5, reactors (10a) and (10b) are separate and are supplied separately from raw material store (11a) with educt glucoside phosphorylase and from raw material store (11b) with product glucoside phosphorylase, sterile filtration preferably first taking place in each case in the device for sterile filtration of the educt glucoside phosphorylase (12a) and in the device for sterile filtration of the product glucoside phosphorylase (12b). In addition, the product mixture leaving the reactor (10b) is preferably fed to a further device for ultrafiltration (29).

### List of reference numerals:

- 1: Raw material store for the reactant for glucose 1 -phosphate
- 2: Raw material store for the educt glucoside
- 3: Raw material store for the phosphate
- 4: Water tank
- 5: Device for softening, treatment (ion exchange, reverse osmosis)
- 6: Device for adjusting the concentration of the water
- 7: Device for adjusting the concentration of the phosphate
- 8: Mixing container
- 9: Device for sterile filtration/heating/UV disinfection
- 10a: Reactor for reaction with catalysis by means of the educt glucoside phosphorylase
- 10b: Reactor for reaction with catalysis by means of the product glucoside phosphorylase
- 11a: Raw material store for educt glucoside phosphorylase
- 11b: Raw material store for product glucoside phosphorylase
- 12a: Device for sterile filtration of the educt glucoside phosphorylase
- 12b: Device for sterile filtration of the product glucoside phosphorylase
- 13: Device for ultrafiltration
- 14: Device for electrodialysis (membrane)
- 15: Device for decolorization
- 16: Raw material store for potassium nitrate (liq.)
- 17: Device for crystallization
- 18: Device for condensation
- 19: Centrifuge/filter press
- 20: Product glucoside <98% purity
- 21: Device for drying
- 22: Product glucoside, pure, dry
- 23: Solution comprising product glucoside, co-product and reactant for glucose 1-phosphate
- 24: Device for SMB (simulated moving bed chromatography)
- 25: Further device for crystallization
- 26: Device for drying
- 27: Co-product, pure, dry
- 28: Further device for condensation
- 29: Further device for ultrafiltration

A further aspect of the invention relates to a composition which comprises product glucoside and is obtainable by the above-described process according to the invention. Preferably, the composition essentially contains no phosphate. It has been found that when separating off the phosphate from the second product composition, e.g. by electrodialysis, the product glucoside, e.g. cellobiose, can be crystallized more efficiently. Furthermore, separating off the phosphate allows its quantitative or nearly quantitative recycling as reactant for step (b). It has been surprisingly found that a quantitative or nearly quantitative recycling of phosphate by means of membrane techniques such as electrodialysis is possible even in presence of high concentrations of monosaccharides (e.g. unreacted glucose, fructose, and the like).

The examples which follow illustrate the invention but are not to be interpreted as being limiting:

### Evaluation Example:

In order to assess the suitability of educt glucoside phosphorylases and product glucoside phosphorylases for the process according to the invention, various candidates were cloned and expressed in *E. coli.*

The genes were cloned into vector pLE1A27 for native expression in *E. coli* (without purification tag). For expression of phosphorylase genes, *E. coli* BL21(DE3) were transformed with the prepared vectors. Expression was performed in LB medium at 25 ml scale in shaking flasks. Once the cultures had reached an optical density (OD₆₀₀) of 0.6-0.8, expressions were induced with 0.1 mM IPTG and the cells were cultivated for 20 h at 30 °C. The wet biomass was treated by ultrasound and cell fragments as well as insolubles were separated from the crude extract by centrifugation. The total amount of produced enzyme was determined by SDS-PAGE-analysis. Further, it was determined what portion was active and dissolved in the cell lysate and what portion was present in inclusion bodies. Furthermore, volume activity of phosphorylase in the crude extract at room temperature was measured.

The results are summarized in the following table:

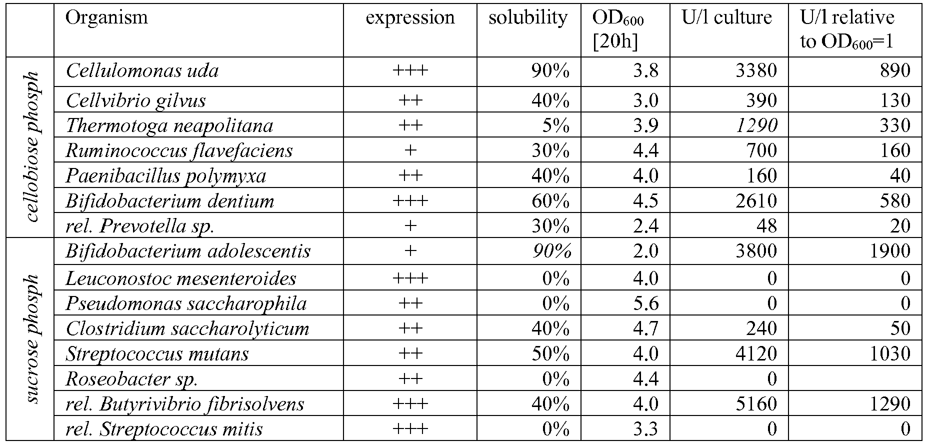

### Example 1 (non-immobilized enzymes, two-stage reaction):

256 g of sucrose are dissolved in 1 liter of water with 48 g of sodium dihydrogen phosphate (NaH₂PO₄) and 57 g of disodium hydrogen phosphate (Na₂HPO₄) or 70 g of dipotassium hydrogen phosphate (K₂HPO₄) and 48 g of potassium dihydrogen phosphate (KH₂PO₄). The process solution is brought to a temperature of 30°C in a stirred vessel reactor, and the reaction is started by addition of 5 kU of sucrose phosphorylase from *Bifidobacterium adolescentis.* The pH value of the process solution is 6.5 and is corrected, if necessary, with phosphoric acid or sodium or potassium hydroxide. The reaction proceeds for 20 hours. The reaction solution is ultrafiltered over a PES (polyethersulfone) membrane with a cut-off of 10 kDa.

Option 1: In order to separate off sucrose, the ultrafiltered process solution is filtered over a nanofiltration membrane and residual sucrose is separated off.

Option 2: In order to retain sucrose as educt, glucose 1-phosphate, monosaccharides and phosphate salts are separated off by an electrodialysis.

The permeate is added equimolarly to glucose 1-phosphate with glucose as reactant in a stirred vessel reactor and brought to the reaction temperature of 30°C (final concentration of glucose 1-phosphate in the first product composition: approximately 0.5 M). The reaction is started by addition of 3 kU of cellobiose phosphorylase from *Cellulomonas uda.* The reaction proceeds at pH 6.5 for 20 hours. When a yield of at least 50% has been reached, the reaction solution is worked up by (optionally multistage) nanofiltration with membranes from GE Desal type NF-DL3840-30D or Koch Membrane Systems type NF-3839 SR3D-NYV or by chromatography, and monosaccharides, such as glucose and fructose, contained in the process solution are thereby separated off.

Ethanol is added to the product stream comprising cellobiose in a stirred vessel reactor, whereby cellobiose is precipitated from the solution in the form of an amorphous solid. The solid is dried on a belt dryer/spin flash dryer. Alternatively, the product stream comprising cellobiose is fed to the crystallization without prior separation of the monosaccharides, and cellobiose is obtained in crystalline form. The crystals are then dried with warm air.

For some applications, the solid is dissolved in water and purified over a chromatography column and decolorized. The product solution comprising cellobiose is dried in a spray dryer. For other applications, the dried solid from the spin flash dryer can be used without further processing or can be converted into a storage-stable compressed product by compaction.

### Reference Example 2 (immobilized enzyme):

10 g of a commercial amino-functionalized carrier (e.g. from Purolite or Resindion) are suspended with 1% (v/v) glutardialdehyde in 40 ml of a 20 mM potassium phosphate buffer (pH 8.0) and incubated for 2 hours with shaking at room temperature. The activated carriers are then washed 2x with 40 ml of a 20 mM potassium phosphate buffer (pH 8.0) and once with 40 ml of a 50 mM potassium phosphate buffer (pH 7.0). 29 kU of the sucrose phosphorylase from *Bifidobacterium adolescentis* or 0.6-1.2 kU of cellobiose phosphorylase from *Cellulomonas uda* were made up to 40 ml in 30 mM potassium phosphate buffer (pH 7.0). This enzyme-buffer solution was incubated for 20 hours with shaking at room temperature. The immobilizates are then washed 3x with 750 mM phosphate buffer (pH 6.5) and finally 1x with 50 mM phosphate buffer. The activity of the immobilizates is determined, and 5 kU of the immobilized sucrose phosphorylase from *Bifidobacterium adolescentis* and 3 kU of cellobiose phosphorylase from *Cellulomonas uda* per liter of the respective substrate solution are used analogously to Example 1 for the first reaction stage. The immobilizates are recovered by sedimentation or filtration. Working up of the product streams and of the end product is likewise carried out analogously to Example 1.

### Example 3: "One-pot" reaction (non-immobilized enzymes, one-stage reaction)

256 g of sucrose are placed in a reaction vessel with 60 g of potassium dihydrogen phosphate (K₂HPO₄), 98 g of dipotassium hydrogen phosphate (KH₂PO₄) and 90 g of glucose, and the mixture is made up to 1 liter with water (735 ml) and dissolved. The process solution is brought to a reaction temperature of 30°C in a stirred vessel reactor, and the reaction is started by addition of 5 kU of sucrose phosphorylase from *Bifidobacterium adolescentis* and 3 kU of cellobiose phosphorylase from *Cellulomonas uda* per liter. The pH value of the process solution is 6.5 and is corrected, if necessary, with phosphoric acid or sodium or potassium hydroxide. The reaction proceeds for about 20 hours. The reaction solution is ultrafiltered over a PES (polyethersulfone) membrane with a cut-off of 10 kDa. Working up of the product streams and of the end product is likewise carried out analogously to Example 1.

## Claims

1. An enzymatic process for the preparation of cellobiose and of fructose from sucrose, wherein the process comprises the steps:
(a) providing a first educt composition which comprises a sucrose phosphorylase, sucrose and phosphate;
(b) reacting at least a portion of the sucrose contained in the first educt composition with at least a portion of the phosphate contained in the first educt composition with catalysis by means of the sucrose phosphorylase, wherein there is obtained a first product composition which comprises fructose and glucose 1-phosphate;
(c) providing a second educt composition which comprises a cellobiose phosphorylase, glucose and at least a portion of the glucose 1-phosphate obtained in step (b); and
(d) reacting the glucose with at least a portion of the glucose 1-phosphate contained in the second educt composition with catalysis by means of the cellobiose phosphorylase, wherein there is obtained a second product composition which comprises the cellobiose and phosphate;
wherein steps (b) and (d) are carried out in a common reactor; and
wherein the sucrose phosphorylase and the cellobiose phosphorylase are neither immobilized on a solid carrier nor enclosed in a matrix.

2. The process according to claim 1, wherein the sucrose phosphorylase, and/or the cellobiose phosphorylase are recycled.

3. The process according to claim 1 or 2, which is carried out in one membrane reactor.

4. The process according to claim 3, wherein the sucrose phosphorylase, and/or the cellobiose phosphorylase cannot pass the membrane of the membrane reactor.

5. The process according to any of the preceding claims, wherein steps (b) and (d) are carried at least partially simultaneously.

6. The process according to any of the preceding claims, wherein the sucrose phosphorylase is the sucrose phosphorylase of the wildtype of a bacterium belonging to the phylum of *Actinobacteria,* or a sucrose phosphorylase differing from said wildtype but comprising an amino acid sequence having at least 80% identity compared to the sucrose phosphorylase of the wildtype of a bacterium belonging to the phylum of *Actinobacteria.*

7. The process according to any of the preceding claims, wherein the cellobiose phosphorylase is the cellobiose phosphorylase of the wildtype of a bacterium belonging to the phylum of *Actinobacteria,* or a cellobiose phosphorylase differing from said wildtype but comprising an amino acid sequence having at least 80% identity compared to the cellobiose phosphorylase of the wildtype of a bacterium belonging to the phylum of *Actinobacteria.*

8. The process according to any of the preceding claims, wherein the concentration of the sucrose in the first educt composition provided in step (a) is at least 0.150 mol/l; preferably at least 0.200 mol/l; more preferably at least 0.500 mol/l.

9. The process according to any of the preceding claims, wherein the concentration of the cellobiose in the second product composition obtained in step (d) is at least 0.075 mol/l; preferably at least 0.150 mol/l; more preferably at least 0.200 mol/l.

10. The process according to any of the preceding claims, wherein step (b) additionally comprises the subsidiary step
(b') separating off at least a portion of the resulting glucose 1-phosphate during the reaction of sucrose to glucose 1-phosphate.

11. The process according to claim 10, wherein in step (b') the glucose 1-phosphate is separated off by electrodialysis and/or crystallization.

12. The process according to any of the preceding claims, which comprises the additional step:
(g) separating off the phosphate obtained in step (d) and optionally returning it to step (a).

13. The process according to any of claim 12, wherein the phosphate is separated off by means of electrodialysis.

14. The process according to claim 12 or 13, wherein at least 50% of the phosphate that is separated off in step (g) is returned to step (a) as at least a portion of the phosphate that is contained in the first educt composition.

15. The process according to any of claims 12 to 14, wherein step (g) is carried out temporally after step (d).

## Patentansprüche

1. Ein enzymatisches Verfahren zur Herstellung von Cellobiose und Fructose aus Saccharose, wobei das Verfahren die Schritte umfasst:
(a) Bereitstellen einer ersten Eduktzusammensetzung, die eine Saccharosephosphorylase, Saccharose und Phosphat umfasst;
(b) Umsetzen mindestens eines Teils der in der ersten Eduktzusammensetzung enthaltenen Saccharose mit mindestens einem Teil des in der ersten Eduktzusammensetzung enthaltenen Phosphats unter Katalyse durch die Saccharosephosphorylase, wobei eine erste Produktzusammensetzung erhalten wird, die Fructose und Glucose-1-phosphat umfasst;
(c) Bereitstellen einer zweiten Eduktzusammensetzung, die eine Cellobiosephosphorylase, Glucose und mindestens einen Teil des in Schritt (b) erhaltenen Glucose-1-phosphats umfasst; und
(d) Umsetzen der Glucose mit mindestens einem Teil des in der zweiten Eduktzusammensetzung enthaltenen Glucose-1-phosphats unter Katalyse durch die Cellobiosephosphorylase, wobei eine zweite Produktzusammensetzung erhalten wird, die die Cellobiose und Phosphat umfasst;
wobei die Schritte (b) und (d) in einem gemeinsamen Reaktor durchgeführt werden; und
wobei die Saccharosephosphorylase und die Cellobiosephosphorylase weder auf einem festen Träger immobilisiert, noch in einer Matrix eingeschlossen sind.

2. Das Verfahren nach Anspruch 1, wobei die Saccharosephosphorylase und/oder die Cellobiosephosphorylase recycelt werden.

3. Das Verfahren nach Anspruch 1 oder 2, das in einem Membranreaktor durchgeführt wird.

4. Das Verfahren nach Anspruch 3, wobei die Saccharosephosphorylase und/oder die Cellobiosephosphorylase die Membran des Membranreaktors nicht passieren können.

5. Das Verfahren nach einem der vorhergehenden Ansprüche, wobei die Schritte (b) und (d) zumindest teilweise gleichzeitig durchgeführt werden.

6. Das Verfahren nach einem der vorhergehenden Ansprüche, wobei die Saccharosephosphorylase die Saccharosephosphorylase des Wildtyps eines zum Stamm der Actinobakterien gehörenden Bakteriums oder eine von dem Wildtyp abweichende Saccharosephosphorylase ist, aber eine Aminosäuresequenz mit einer Identität von mindestens 80% gegenüber der Saccharosephosphorylase des Wildtyps eines zum Stamm der Actinobakterien gehörenden Bakteriums umfasst.

7. Das Verfahren nach einem der vorhergehenden Ansprüche, wobei die Cellobiosephosphorylase die Cellobiosephosphorylase des Wildtyps eines zum Stamm der Actinobakterien gehörenden Bakteriums oder eine von dem Wildtyp abweichende Cellobiosephosphorylase ist, aber eine Aminosäuresequenz mit einer Identität von mindestens 80% gegenüber der Cellobiosephosphorylase des Wildtyps eines zum Stamm der Actinobakterien gehörenden Bakteriums umfasst.

8. Das Verfahren nach einem der vorhergehenden Ansprüche, wobei die Konzentration der Saccharose in der in Schritt (a) vorgesehenen ersten Eduktzusammensetzung mindestens 0,150 mol/L; bevorzugt mindestens 0,200 mol/L; besonders bevorzugt mindestens 0,500 mol/L beträgt.

9. Das Verfahren nach einem der vorhergehenden Ansprüche, wobei die Konzentration der Cellobiose in der in Schritt (d) erhaltenen zweiten Produktzusammensetzung mindestens 0,075 mol/L; bevorzugt mindestens 0,150 mol/L; besonders bevorzugt mindestens 0,200 mol/L beträgt.

10. Das Verfahren nach einem der vorhergehenden Ansprüche, wobei Schritt (b) zusätzlich den Nebenschritt
(b') Abtrennen mindestens eines Teils des resultierenden Glucose-1-phosphats während der Reaktion von Saccharose zu Glucose-1-phosphat umfasst.

11. Das Verfahren nach Anspruch 10, wobei in Schritt (b') das Glucose-1-phosphat durch Elektrodialyse und/oder Kristallisation abgetrennt wird.

12. Das Verfahren nach einem der vorhergehenden Ansprüche, das den zusätzlichen Schritt:
(g) Abtrennen des in Schritt (d) erhaltenen Phosphats und gegebenenfalls Rückführung in Schritt (a) umfasst.

13. Das Verfahren nach Anspruch 12, wobei das Phosphat durch Elektrodialyse abgetrennt wird.

14. Das Verfahren nach Anspruch 12 oder 13, wobei mindestens 50% des in Schritt (g) abgetrennten Phosphats als mindestens ein Teil des Phosphats, das in der ersten Eduktzusammensetzung enthalten ist, in Schritt (a) zurückgeführt wird.

15. Das Verfahren nach einem der Ansprüche 12 bis 14, wobei Schritt (g) zeitlich nach Schritt (d) durchgeführt wird.

## Revendications

1. Procédé enzymatique pour la préparation de cellobiose et de fructose à partir de saccharose, le procédé comprenant les étapes de :
(a) mise à disposition d'une première composition d'éduit comprenant une saccharose phosphorylase, du saccharose et du phosphate ;
(b) mise en réaction d'au moins une partie du saccharose contenu dans la première composition d'éduit avec au moins une partie du phosphate contenu dans la première composition d'éduit catalysée par la saccharose phosphorylase, une première composition de produit étant obtenue, comprenant du fructose et du glucose-1-phosphate ;
(c) mise à disposition d'une deuxième composition d'éduit comprenant une cellobiose phosphorylase, du glucose et au moins une partie du glucose-1-phosphate obtenu dans l'étape (b) ; et
(d) mise en réaction du glucose avec au moins une partie du glucose-1-phosphate contenu dans la deuxième composition d'éduit catalysée par la cellobiose phosphorylase, une deuxième composition de produit étant obtenue qui comprend le cellobiose et du phosphate ;
les étapes (b) et (d) étant mises en oeuvre dans un réacteur commun ; et
la saccharose phosphorylase et la cellobiose phosphorylase n'étant ni immobilisées sur un vecteur solide ni confinées dans une matrice.

2. Procédé selon la revendication 1, la saccharose phosphorylase et/ou la cellobiose phosphorylase étant recyclée(s).

3. Procédé selon la revendication 1 ou 2, mis en oeuvre dans un réacteur à membrane.

4. Procédé selon la revendication 3, la saccharose phosphorylase et/ou la cellobiose phosphorylase ne pouvant pas traverser la membrane du réacteur à membrane.

5. Procédé selon l'une quelconque des revendications précédentes, les étapes (b) et (d) étant mises en oeuvre au moins en partie simultanément.

6. Procédé selon l'une quelconque des revendications précédentes, la saccharose phosphorylase étant la saccharose phosphorylase du type sauvage d'une bactérie appartenant au phylum *Actinobacteria* ou une saccharose phosphorylase différente dudit type sauvage mais comprenant une séquence d'acides aminés possédant au moins 80% d'identité par rapport à la saccharose phosphorylase du type sauvage d'une bactérie appartenant au phylum *Actinobacteria.*

7. Procédé selon l'une quelconque des revendications précédentes, la cellobiose phosphorylase étant la cellobiose phosphorylase du type sauvage d'une bactérie appartenant au phylum *Actinobacteria* ou une cellobiose phosphorylase différente dudit type sauvage mais comprenant une séquence d'acides aminés possédant au moins 80% d'identité par rapport à la cellobiose phosphorylase du type sauvage d'une bactérie appartenant au phylum *Actinobacteria.*

8. Procédé selon l'une quelconque des revendications précédentes, la concentration en saccharose dans la première composition d'éduit mise à disposition dans l'étape (a) étant d'au moins 0,150 mole/l ; préférablement d'au moins 0,200 mole/l ; plus préférablement d'au moins 0,500 mole/l.

9. Procédé selon l'une quelconque des revendications précédentes, la concentration en cellobiose dans la deuxième composition de produit obtenue dans l'étape (d) étant d'au moins 0,075 mole/l ; préférablement d'au moins 0,150 mole/l ; plus préférablement d'au moins 0,200 mole/1.

10. Procédé selon l'une quelconque des revendications précédentes, l'étape (b) comprenant de plus l'étape supplémentaire de (b') séparation d'au moins une partie du glucose-1-phosphate obtenu pendant la réaction du saccharose en glucose-1-phosphate.

11. Procédé selon la revendication 10, le glucose-1-phosphate dans l'étape (b') étant séparé par électrodialyse et/ou cristallisation.

12. Procédé selon l'une quelconque des revendications précédentes, comprenant l'étape supplémentaire de :
(g) séparation du phosphate obtenu dans l'étape (d) et renvoi éventuel de celui-ci dans l'étape (a) .

13. Procédé selon la revendication 12, le phosphate étant séparé par électrodialyse.

14. Procédé selon la revendication 12 ou 13, au moins 50% du phosphate séparé dans l'étape (g) étant renvoyé à l'étape (a) en tant qu'au moins une partie du phosphate contenu dans la première composition d'éduit.

15. Procédé selon l'une quelconque des revendications 12 à 14, l'étape (g) étant mise en oeuvre dans le temps après l'étape (d).


### SEQUENCE LISTING

<110> Pfeifer & Langen GmbH & Co. KG
<120> Process for the enzymatic preparation of a product glucoside and of a co-product from an educt glucoside
<130> PFL0003-WO
<150> EP14184301
   <151> 2014-09-10
<150> EP14190891
   <151> 2014-10-29
<160> 3
<170> PatentIn version 3.5
<210> 1
   <211> 504
   <212> PRT
   <213> Bifidobacterium adolescentis
<400> 1
<210> 2
   <211> 822
   <212> PRT
   <213> Cellulomonas uda
<400> 2
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Gln Thr Asn Tyr Gln Arg Asn Leu Ser Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asp His Tyr Ala Val Phe Ala Val Asn Thr Gln Ala Glu
           195                 200                 205
   Gly Phe Asp Thr Asp Arg Asp Thr Phe Val Gly Ala Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Leu Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 3
   <211> 822
   <212> PRT
   <213> artificial
<220>
   <223> modified wildtype sequence
<400> 3
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Val Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Val
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
               755                 760                 765
   Thr Val Thr Arg Val Ala Arg Gly Ala Thr Tyr Glu Ile Thr Val Thr
       770                 775                 780
   Asn Ser Gly Val Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
                   820